# EUROPEAN PATENT APPLICATION

(11) **EP 1 265 072 A1**
(43) Date of publication of application: **11.12.2002**
(21) Application number: 01306842.4
(22) Date of filing: 10.08.2001
(51) Int. Cl.: G01N 33/68, C07K 1/12

(54) **Method for characterising polypeptides**

(30) Priority: 07.06.2001 EP 01304975
(71) Applicant: Xzillion GmbH & CO.KG, 65926 Frankfurt am Main (DE)
(72) Inventor: The designation of the inventor has not yet been filed
(74) Representative: Hill, Christopher Michael

(57) **Abstract**

Provided is a method for characterising a polypeptide or a population of polypeptides, which method comprises the steps of:
(a) Optionally reducing disulphide linkages in the polypeptides, if they are present, and capping free thiols in the polypeptides, if they are present;
(b) contacting a sample comprising one or more polypeptides with a sequence specific cleavage reagent which cleaves one or more polypeptides on the C-terminal side of a lysine residue to produce peptide fragments;
(c) optionally deactivating the cleavage reagent;
(d) contacting the sample with a lysine reactive agent to cap ε-amino groups;
(e) removing those peptides having capped ε-amino groups; and
(f) recovering the C-terminal peptides

## Description

This invention relates to methods of isolating a single C-terminal peptide from each protein in a population. This invention further relates to the use of the above methods in methods of determining the expression of proteins in a tissue, cell type, or sub-cellular compartment or in analysing large protein complexes. This invention also relates to the use of the above methods of C-terminal peptide isolation for the analysis of chromatographically-separated protein fractions or mixtures of proteins isolated by affinity capture.

Techniques for profiling proteins, that is to say cataloguing the identities and quantities of proteins in a tissue, are not well developed in terms of automation or high throughput. A typical method of profiling a population of proteins is by two-dimensional electrophoresis (R.A. Van Bogelen., E.R. Olson, "Application of two-dimensional protein gels in biotechnology", Biotechnol Annu. Rev., 1, 69-103, 1995). In this method, a protein sample extracted from a biological sample is separated on a narrow gel strip. This first separation usually separates proteins on the basis of their iso-electric point. The entire gel strip is then laid against one edge of a rectangular gel. The separated proteins in the strip are then electrophoretically separated in the second gel on the basis of their size. This technology is slow and very difficult to automate. It is also relatively insensitive in its simplest embodiments. A number of improvements have been made to increase resolution of proteins by 2-D gel electrophoresis and to improve the sensitivity of the system. One approach to improve the sensitivity of 2-D gel electrophoresis and its resolution is to analyse the protein in specific spots on the gel by mass spectrometry (Jungblut P, Thiede B. "Protein identification from 2-D gels by MALDI mass spectrometry." Mass Spectrom. Rev. 16, 145-162, 1997. One example of a mass spectrometry method is in-gel tryptic digestion followed by analysis of the tryptic fragments by mass spectrometry to generate a peptide mass fingerprint. If sequence information is required, tandem mass spectrometry analysis can be performed.

More recently attempts have been made to exploit mass spectrometry to analyse whole proteins that have been fractionated by liquid chromatography or capillary electrophoresis (Dolnik V. "Capillary zone electrophoresis of proteins.", Electrophoresis 18, 2353-2361, 1997). In-line systems exploiting capillary electrophoresis mass spectrometry have been tested. The analysis of whole proteins by mass spectrometry, however, suffers from a number of difficulties. The first difficulty is the analysis of the complex mass spectra resulting from multiple ionisation states accessible by individual proteins. The second major disadvantage is that the mass resolution of mass spectrometers is at present quite poor for high molecular weight species, i.e. for ions that are greater than about 4 kilodaltons (kDa) in mass, so resolving proteins that are close in mass is difficult. A third disadvantage is that further analysis of whole proteins by tandem mass spectrometry is difficult as the fragmentation patterns for whole proteins are extremely complex and difficult to interpret.

As a result of the difficulties of analysing whole proteins, techniques that rely on the analysis of peptides from proteins are preferred. Peptide mass fingerprinting has been used in the analysis of gel separated proteins as described above. However, this process is adequate only for the analysis of individual proteins or very simple mixtures of proteins. A typical protein will give rise to from twenty to thirty peptides after cleavage with trypsin. The pattern of peptide masses is useful for identifying single proteins, but the complexity of the mass spectrum of the trypsin digest of a mixture of proteins rapidly rises in complexity as the number of proteins in the mixture increases. This increases the chance that a peptide mass is assigned incorrectly to a protein, thus limiting the number of proteins that may be analysed simultaneously. As a result new protein characterisation methods are being developed in which specific peptides are isolated from each protein in a mixture.

Nature Biotechnology 17, 994-999 (1999) discloses the use of'isotope encoded affinity tags' for the capture of peptides from proteins, to allow protein expression analysis. In this article, the authors describe the use of a biotin linker, which is reactive to thiols, for the capture peptides with cysteine in them. A sample of protein from one source is reacted with the biotin linker and cleaved with an endopeptidase. The biotinylated cysteine-containing peptides can then be isolated on avidinated beads for subsequent analysis by mass spectrometry. Two samples can be compared quantitatively by labelling one sample with the biotin linker and labelling the second sample with a deuterated form of the biotin linker. Each peptide in the samples is then represented as a pair of peaks in the mass spectrum where the relative peak heights indicate their relative expression levels.

This 'isotope encoding' method has a number of limitations. A first limitation is the reliance on the presence of thiols in a protein - many proteins do not have thiols while others have several. In a variation on this method, linkers may be designed to react with other side chains, such as amines. However, since many proteins contain more than one lysine residue, multiple peptides per protein would generally be isolated in this approach. It is likely that this would not reduce the complexity of the sample sufficiently for analysis by mass spectrometry. A sample that contains too many species is likely to suffer from 'ion suppression', in which certain species ionise preferentially over other species which would normally appear in the mass spectrum in a less complex sample. In general, capturing proteins by their side chains is likely to give either too many peptides per protein or certain proteins will be missed altogether.

The second limitation of this approach is the method used to compare the expression levels of proteins from different samples. Labelling each sample with a different isotope variant of the affinity tag results in an additional peak in the mass spectrum for each peptide in each sample. This means that if two samples are analysed together there will be twice as many peaks in the spectrum. Similarly, if three samples are analysed together, the spectrum will be three times more complex than for one sample alone. It is clear that this approach will be limited, since the ever increasing numbers of peaks will increase the likelihood that two different peptides will have overlapping peaks in the mass spectrum.

A further limitation, which is reported by the authors of the above paper, is the mobility change caused by the tags. The authors report that peptides labelled with the deuterated biotin tag elute slightly after the same peptide labelled with the undeuterated tag.

Published international patent application WO 98/32876 discloses methods of profiling a population of proteins by isolating a single peptide from one terminus of each protein in the population. In a first aspect the invention comprises the steps of:
1. capturing a population of proteins onto a solid phase support by one terminus of each protein in the population;
2. cleaving the captured proteins with a sequence specific cleavage agent;
3. washing away peptides generated by the cleavage agent not retained on the solid phase support;
4. releasing the terminal peptides retained on the solid phase support; and
5. analysing the released terminal peptides, preferably identifying and quantifying each peptide in the mixture. The analysis is preferably performed by mass spectrometry.

In this application, the C-terminus is discussed as being more preferable as the terminus by which to capture a population of proteins, since the N-terminus is often blocked. In order to capture a population of proteins by the C-terminus, the C-terminal carboxyl group must be distinguished from other reactive groups on a protein and must be reacted specifically with a reagent that can effect immobilisation. In many C-terminal sequencing chemistries the C-terminal carboxyl group is activated to promote formation of an oxazolone group at the C-terminus. During the activation of the C-terminal carboxyl, side chain carboxyls are also activated, but these cannot form an oxazolone group. It has been reported that the C-terminal oxazolone is less reactive to nucleophiles under basic conditions than the activated side-chain carboxyls, offering a method of selectively capping the side chain carboxyl groups (V. L. Boyd et al., Methods in Protein Structure Analysis: 109-118, Plenum Press, Edited M. Z. Atassi and E. Appella, 1995). Other more reactive side chains can be capped prior to the activation of the carboxyls using a variety of conventional reagents. In this way all reactive side chains can be capped and the C-terminus can be specifically labelled.

EP A 0 594 164 and EP B 0 333 587 describe methods of isolating a C-terminal peptide from a protein in a method to allow sequencing of the C-terminal peptide using N-terminal sequencing reagents. In this method the protein of interest is digested with an endoprotease, which cleaves at the C-terminal side of lysine residues. The resultant peptides are reacted with diisothiocyanato (DITC) polystyrene which reacts with all free amino groups. N-terminal amino groups that have reacted with the DITC polystyrene can be cleaved with trifluoroacetic acid (TFA) thus releasing the N-terminus of all peptides. The epsilon-amino group of lysine is not cleaved however and all non-terminal peptide are thus retained on the support and only C-terminal peptides are released. According to this patent the C-terminal peptides are recovered for micro-sequencing.

Anal. Biochem. 132, 384-388 (1983) and DE A 4344425 (1994) describe methods of isolating an N-terminal peptide from a protein by reacting the protein with a capping reagent which will cap any free amino groups in the protein. The protein is then cleaved, and if trypsin is used cleavage occurs only at arginine residues. Cleavage with trypsin thus exposes α-amino groups in the non-N-terminal peptides. In the first disclosure (Anal. Biochem.) the α-amino groups are reacted with dinitrofluorobenzene (DNF) which allows the non-N-terminal peptides to be captured by affinity chromatography onto a polystyrene resin while the N-terminal peptides flow through unimpeded. In DE A 4344425, the epsilon amino groups are reacted with an acylating agent prior to cleavage. After cleavage in this method, the α-amino groups on the non-N-terminal peptides are reacted with an amine reactive solid support such as diisothiocyanato glass, leaving the N-terminal peptides free in solution.

The main drawback of all of these peptide isolation methods is the use of conventional amine modification reagents which tend to be unstable in aqueous conditions at the pH needed for lysine modification. As a result, large excesses of reagent need to be used which can lead to side-reactions particularly with histidine residues. The Anal. Biochem. method also requires that the DNF groups be removed from histidine and tyrosine by thiolysis prior to isolating the N terminal peptide, if the N terminal peptide contains these groups. This additional step requires extra effort and may not go to completion. In the Anal. Chem. disclosure the protein and terminal peptides are not analysed by mass spectrometry and so it is not possible to know whether the capping of the lysine epsilon amino groups goes to completion.

It is an aim of this invention to solve the problems associated with the known methods described above. It is thus an aim of this invention to provide improved methods for isolating a single C-terminal peptide from each protein in a mixture of polypeptides using protein reactive reagents that are stable in water, selective for lysine and that work under mild reaction conditions without degradation of the reagents. It is a further aim that these reactions go substantially to completion in a relatively short time, e.g. in a few hours.

Accordingly, the present invention provides a method for characterising a polypeptide or a population of polypeptides, which method comprises the steps of:
(a) contacting a sample comprising one or more polypeptides with a sequence specific cleavage reagent which cleaves one or more polypeptides on the C-terminal side of a lysine residue to produce peptide fragments;
(b) optionally deactivating the cleavage reagent;
(c) contacting the sample with a lysine reactive agent to cap ε-amino groups;
(d) removing those peptide fragments having capped ε-amino groups; and
(e) recovering the C-terminal peptide fragments.

The steps (a) and (c) of the method of the present invention can be carried out in any order, provided that the C-terminal peptide fragments can be isolated. Thus, in some embodiments the peptides can be cleaved prior to capping, or in other embodiments, the residues can be capped whilst still forming part of a polypeptide, which polypeptide is subsequently cleaved. In the latter embodiments, the cleavage reagent is capable of cleaving on the C-terminal side of lysine residues even after these residues have been capped.

The peptide fragments comprising capped ε-amino groups are preferably removed by capturing these fragments, e.g. on a solid phase. In this embodiment, the lysine reactive agent is a lysine selective capture agent. Selective capture may be achieved by attaching a capture group to the lysine reactive agent (such as biotin), which ensures that the agent along with its capped peptide fragment attaches to a solid phase (such as an avidinated solid phase) after capping has occurred. In an alternative embodiment, the lysine reactive agent may be attached to a solid phase before the capping takes place, so that the peptide fragments are captured onto the solid phase by the capping reaction itself.

The capped fragments can thus be removed from the sample by separating the sample from the solid phase, leaving the C-terminal fragments as the only peptide fragments left in the sample. These C-terminal fragments may then be analysed to determine the polypeptides present in the original sample.

This method allows lower concentrations of the reagents to be used at higher pH. Both of these factors have been found by the inventors to improve the selectivity and completeness of lysine reactions. In the following description, lysine amino groups will be referred to as epsilon amino (ε-amino) groups.

The lysine reactive agent is preferably a hindered Michael reagent. A Michael reagent has a general formula as below:

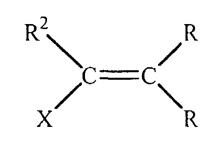

In the above formula, X is an electron withdrawing group that is capable of stabilising a negative charge. The functional group -X is preferably selected from those listed in Table 1 below:

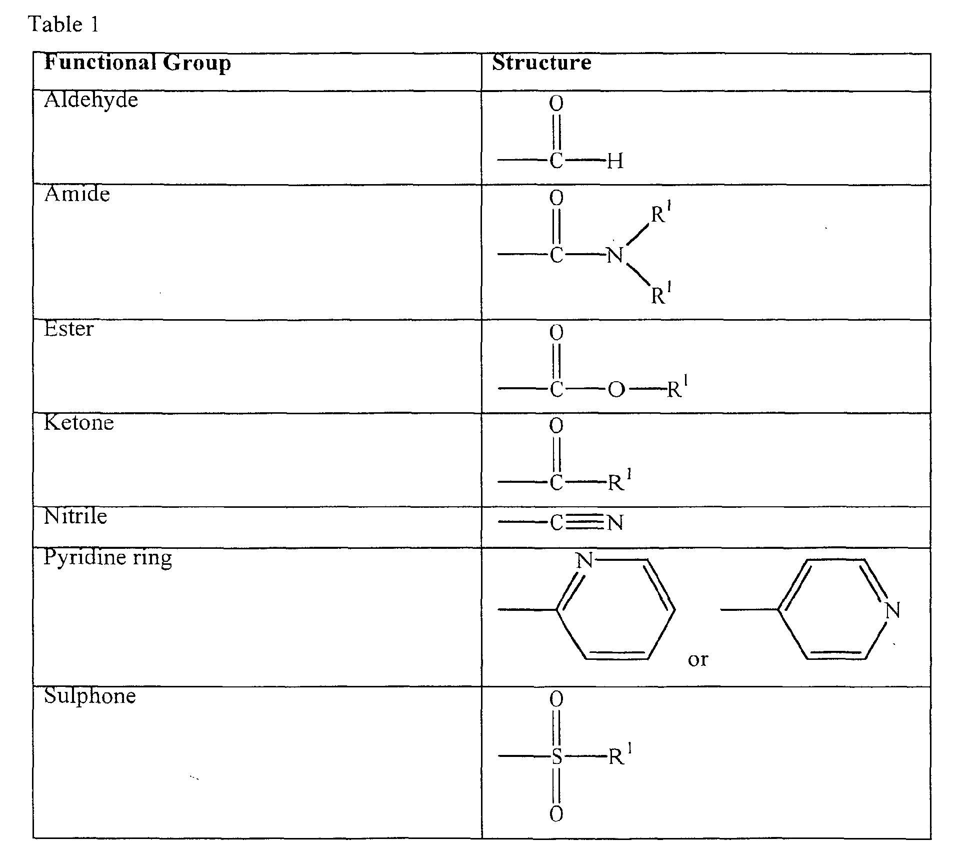

Where R¹ may be any alkyl or aromatic group but is preferably an electron withdrawing group and more preferably a cyclic or heterocylic aromatic ring or fused ring. Preferably the ring structure is electron withdrawing. More specifically R¹ is preferably a small ring or fused ring such as a phenyl, pyridyl, naphthyl or quinolyl ring structure. Preferred ring structures are substituted with appropriate electron withdrawing groups such as halogens like fluorine or nitro groups. Preferred ring structures promote water solubility, such as pyridyl and naphthyl rings. If -X is an amide, then one or both of the R¹ groups may be a hydrogen atom. If -X is a nitrile, preferred compounds include crotonitriles such as trifluorocrotonitrile. R¹ may additionally comprise a linker to an affinity capture functionality, such as biotin, or a linker to a solid phase support.

In the formula above R² is either a hydrogen atom or it may comprise an electron-withdrawing group and/or a linker to an affinity capture functionality or a linker to a solid phase support. Further specific groups that R² may be are listed below in the definition of the group Sub.

To be a 'hindered' Michael reagent according to this invention, at least one of the R groups is not hydrogen and is considered to be a sterically hindering group. At least one R group may comprise an alkyl or aromatic group such as a methyl or phenyl group. More preferably at least one of the R groups is electron-withdrawing and may comprise a halogen atom or a halogenated alkyl group, such as fluoromethyl, difluoromethyl or trifluoromethyl group or a phenyl ring with electron withdrawing substituents such as halogen or nitro groups. In addition, one R-group may comprise a linker to an affinity capture functionality, such as biotin, or a linker to a solid phase support. Conversely to be an 'unhindered' Michael reagent in the context of this invention, both R groups would be hydrogen.

In a preferred embodiment, one (and more preferably only one) of the X-, R-, R¹- and R²-groups comprises a linker to an affinity capture functionality, such as biotin, or a linker to a solid phase support.

In some embodiments, the X group may be joined to one of the R groups to form a ring. Preferred compounds of this type include maleimides of the formula:

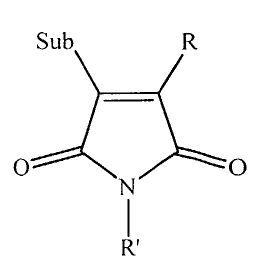

Where R has the same meaning as above and R' is a hydrocarbon group or an electron donating group. Preferably R comprises an alkyl group or aryl group and particularly preferably R comprises a C₁-C₆ alkyl group, such as a methyl or ethyl group.

The group Sub in the above formulae is not particularly limited, provided that the Michael agent is capable of reacting with an ε-amino group. The group is generally a group R² as defined above, and more specifically in preferred embodiments of the invention, Sub comprises a hydrocarbon group such as an alkyl or aryl group or an electron withdrawing group, such as a cyano group (-CN), or a halogen (F, Cl, Br, I) or halogen-containing group. In the most preferred embodiments, Sub comprises a hydrogen, or a C₁-C₆ alkyl group, such as a methyl or ethyl group. A particularly preferred compound is one in which Sub and R are both H and R' comprises a methyl group or an ethyl group.

In the context of this invention, the term lysine-selective reagent refers to the ability of the reagent to discriminate between the epsilon-amino group of lysine and the alpha-amino groups of all amino acids. It is also preferred that the reagents of this invention do not react with other side chain functionalites such as the imidazole ring of histidine, the guanidino group of arginine and hydroxyl functionalities found in serine, threonine and tyrosine.

In the context of this invention, the term capture reagent refers to the ability of the reagent to capture molecules onto a solid support. Thus, as mentioned above, the capture reagent may comprise a reactive functionality linked covalently to a solid phase support. or it may comprise a reactive functionality linked to functionality that can be chemically linked to a solid phase support or it may comprise a reactive functionality linked to an affinity capture functionality, which can be captured to a solid support by interaction with a specific ligand that is linked to the solid support.

The various aspects of this invention will now be discussed in more detail below.

In one embodiment of this invention there is provided a method of isolating a population of C-terminal peptides from a sample of polypeptides comprising the steps of:
1. digesting the sample of polypeptides completely with a sequence specific cleavage reagent that cleaves at the amide bond on the C-terminal side of lysine residues;
2. capturing all non-C-terminal peptides by contacting the resultant capped peptides with a lysine selective capture reagent; and
3. recovering the C-terminal peptides left in solution, which should not have a free epsilon amine to react with a solid support or capture reagent.

In this and other embodiments of the present invention, a further optional step may also be carried out in case disulphide linkages are present This step involves reducing disulphide linkages in the polypeptides, and capping resultant free thiols (and/or free thiols initially present) in the polypeptides. If desired, this step may be carried out prior to digesting the sample with the cleavage agent, e.g.:
1. optionally reducing disulphide linkages in the polypeptides, if they are present, and capping free thiols in the polypeptides if they are present.
2. digesting the sample of polypeptides completely with a sequence specific cleavage reagent that cleaves at the amide bond on the C-terminal side of lysine residues;
3. capturing all non-C-terminal peptides by contacting the resultant capped peptides with a lysine selective capture reagent; and
4. recovering the C-terminal peptides left in solution, which should not have a free epsilon amine to react with a solid support or capture reagent. In a further aspect, this invention provides a method for determining the expression profile of a sample, which method comprises characterising one or more mixtures of polypeptides according to a method as defined above. Thus, this aspect of the invention provides a method of determining the expression profile of at least one mixture of polypeptides, and is a method to identify and preferably also to quantify each polypeptide in the mixture. This method preferably comprises the following steps:
   1. isolating terminal peptides according to the first embodiments of this invention from at least one mixture of polypeptides;
   2. optionally labelling the free alpha amino group of the recovered C-terminal peptides from each sample with a different mass marker;
   3. optionally separating the C-terminal peptides electrophoretically or chromatographically;
   4. detecting the peptides by mass spectrometry.

In a yet further aspect, this invention provides a lysine selective protein labelling reagent that comprises a thiol and amino reactive hindered alkenyl sulphone compounds with the formula:

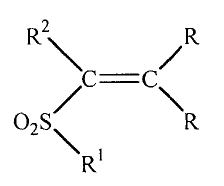

Where R¹ may be any alkyl or aromatic group but is preferably an electron withdrawing group and more preferably a cyclic or heterocylic aromatic ring or fused ring. Preferably the ring structure is electron withdrawing. More specifically R¹ is preferably a small ring or fused ring such as a phenyl, pyridyl, naphthyl or quinolyl ring structure. Preferred ring structures are substituted with appropriate electron withdrawing groups such as halogens like fluorine or nitro groups. Preferred ring structures promote water solubility, such as pyridyl and naphthyl rings. If -X is an amide, then one or both of the R¹ groups may be a hydrogen atom. If -X is a nitrile, preferred compounds include crotonitriles such as trifluorocrotonitrile. R¹ may additionally comprise a linker to an affinity capture functionality, such as biotin, or a linker to a solid phase support.

In the formula above R² is either a hydrogen atom or it may comprise an electron-withdrawing group and/or a linker to an affinity capture functionality or a linker to a solid phase support.

Preferably one and more preferably, only one of the X-, R-, R¹ and R² groups comprises a linker to an affinity capture functionality, such as biotin, or a linker to a solid phase support.

The invention will now be described in more detail by way of example only, with reference to the following Figures:
Figure 1 shows a preferred hindered alkenyl sulphone capture reagent for use with this invention - a synthetic procedure for the production of this reagent is described in example 2;
Figure 2a shows the first page of an illustration of one embodiment of the first aspect of this invention using Calcitonin H and Calcitonin S as examples;
Figure 2b shows the second page of an illustration of one embodiment of the first aspect of this invention using Calcitonin H and Calcitonin S as examples;
Figure 2c shows the third page of an illustration of one embodiment of the first aspect of this invention using Calcitonin H and Calcitonin S as examples;
Figure 2d shows the fourth page of an illustration of one embodiment of the first aspect of this invention using Calcitonin H and Calcitonin S as examples;
Figure 3a shows the first page of an illustration of a second embodiment of the first aspect of this invention using Calcitonin H and Calcitonin S as examples;
Figure 3b shows the second page of an illustration of a second embodiment of the first aspect of this invention using Calcitonin H and Calcitonin S as examples;
Figure 3c shows the third page of an illustration of a second embodiment of the first aspect of this invention using Calcitonin H and Calcitonin S as examples; and
Figure 4 shows shows a thin layer chromatography plate revealing the results of an experiment that uses polystyrene bound maleimide to remove lysine containing peptides from a mixture of peptides. This experiment was done under aqueous conditions;
Figure 5 shows a thin layer chromatography plate revealing the results of an experiment that uses polystyrene bound maleimide to remove lysine containing peptides from a mixture of peptides. This experiment was done under non-aqueous conditions; and
Figure 6 shows a thin layer chromatography plate revealing the results of an experiment that uses polystyrene bound maleimide to remove lysine containing peptides from a mixture of peptides. This experiment was done to determine how much water the maleimide resin can tolerate.

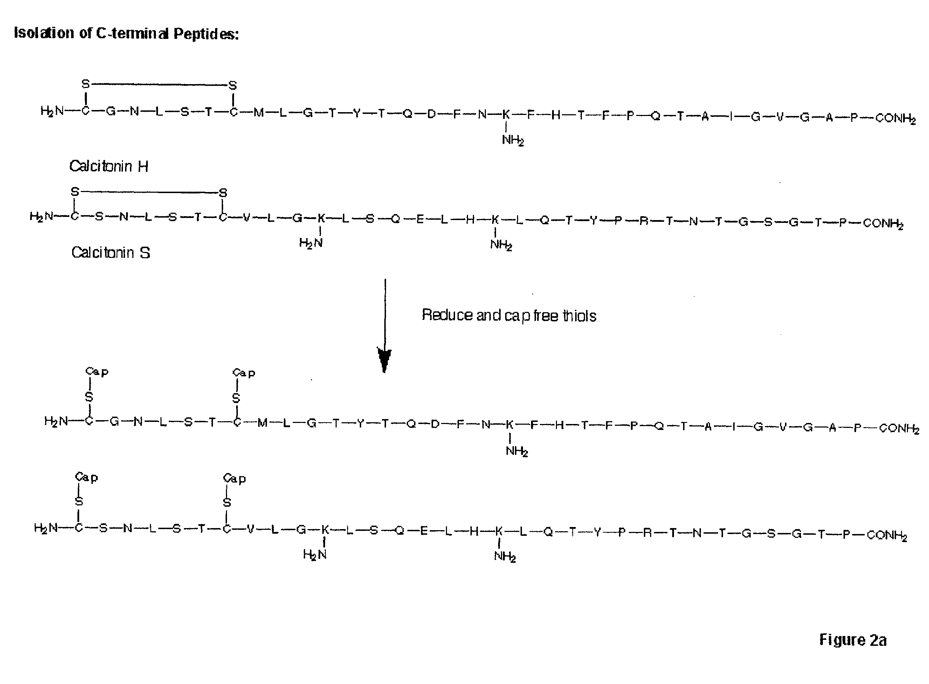

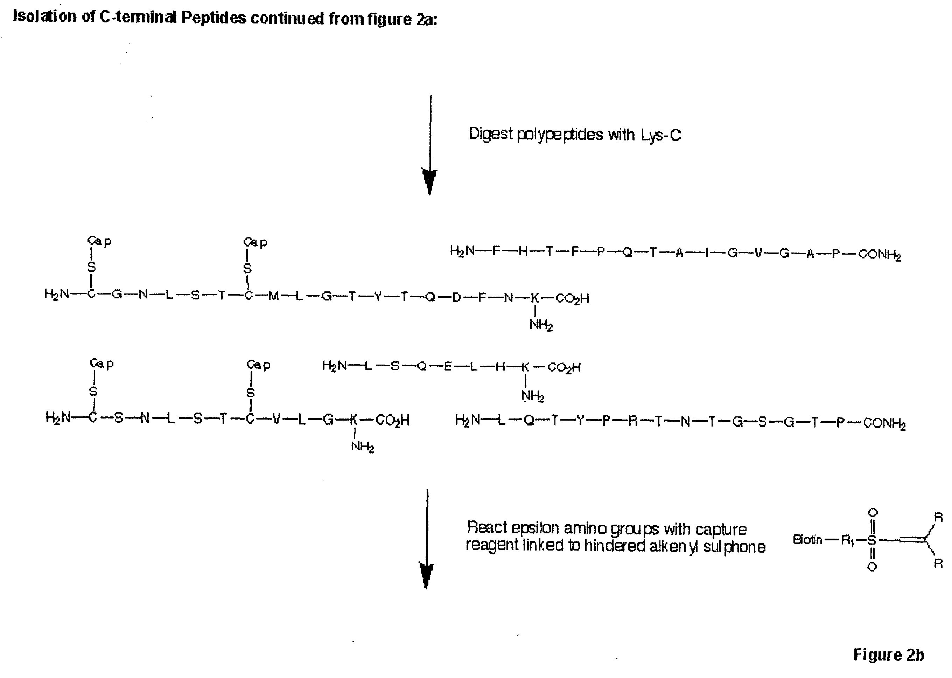

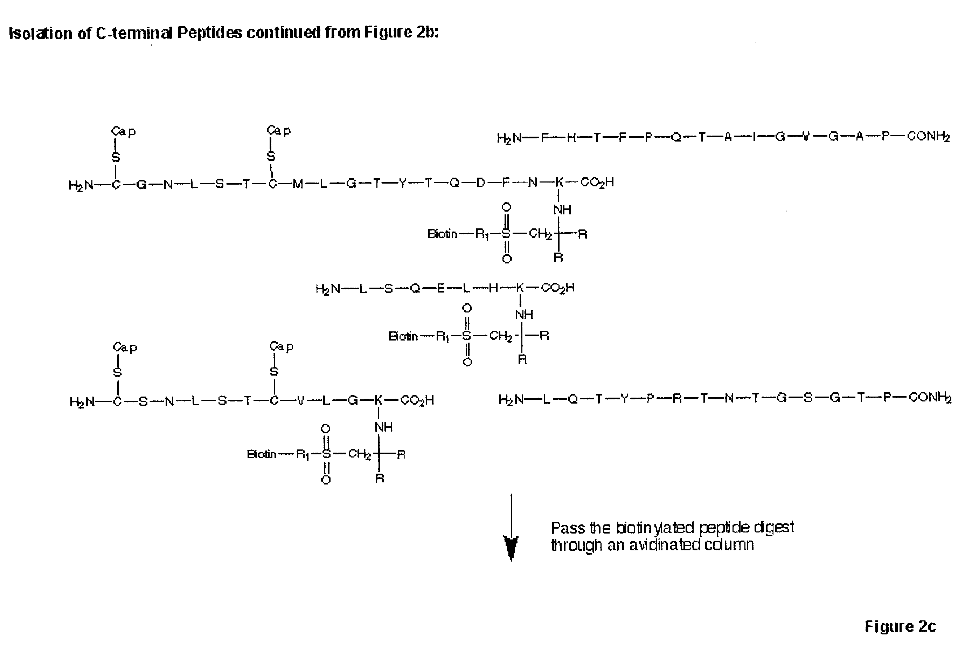

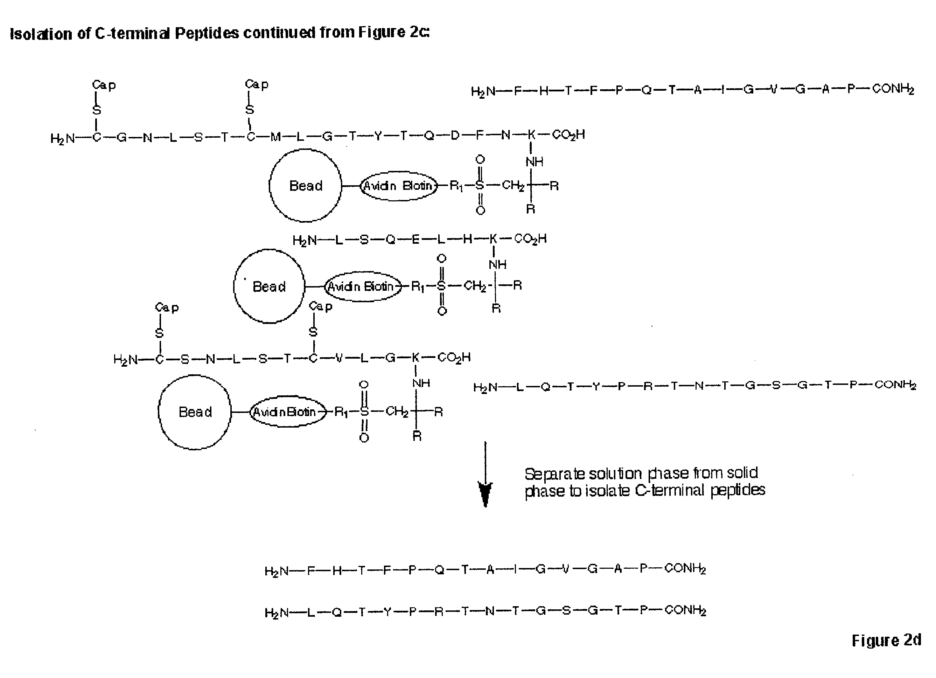

Figures 2a, 2b, 2c and 2d will now be described in more detail. Figures 2a to 2d illustrate an embodiment of the first aspect of this invention, which provides a method of isolating a population of C-terminal peptides from a sample of polypeptides. Figure 2a illustrates an optional, but preferable, first step of this process in which two short polypeptides are reduced and free thiols are capped. Two polypeptides, rather than a complex mixture, are shown, Calcitonin H and Calcitonin S, for ease of illustration.

Figure 2b illustrates the second step of this embodiment of the invention in which the polypeptides are cleaved with a sequence specific cleavage reagent that cleaves at the amide bond on the C-terminal side of lysine residues. In the figure this step is performed with Lys-C. The cleavage reaction generates new free alpha-amino groups in the C-terminal product peptides of each cut.

Figure 2c illustrates the third step of this embodiment of the invention in which the epsilon amino groups in the cleaved peptides are reacted with a capture reagent. In the figure this reagent comprises the affinity capture agent, biotin, linked to a hindered alkenyl sulphone reagent, which reacts selectively with lysine. Since all non-C-terminal peptides have a free epsilon amino group, these peptides will react with the capture reagent. The C-terminal peptides will not be captured as they have no lysine groups.

Figure 2d illustrates the final step in this embodiment of the invention in which the C-terminal peptides are separated from non-C-terminal peptides by passing the solution phase through an affinity column. The resin in the column is derivitised with avidin, a highly selective counter-ligand for biotin. The C-terminal peptides, which are not biotinylated, remain in solution and can be analysed further. Note that cleavage with Lys-C leaves a free alpha amino group available in each C-terminal peptide. This group can be reacted with a label if desired.

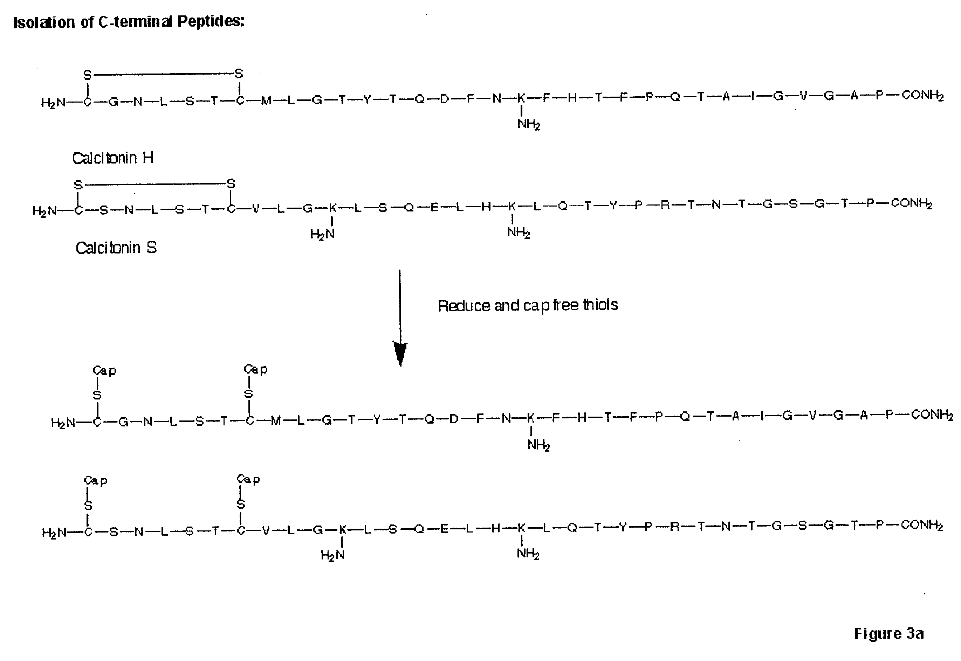

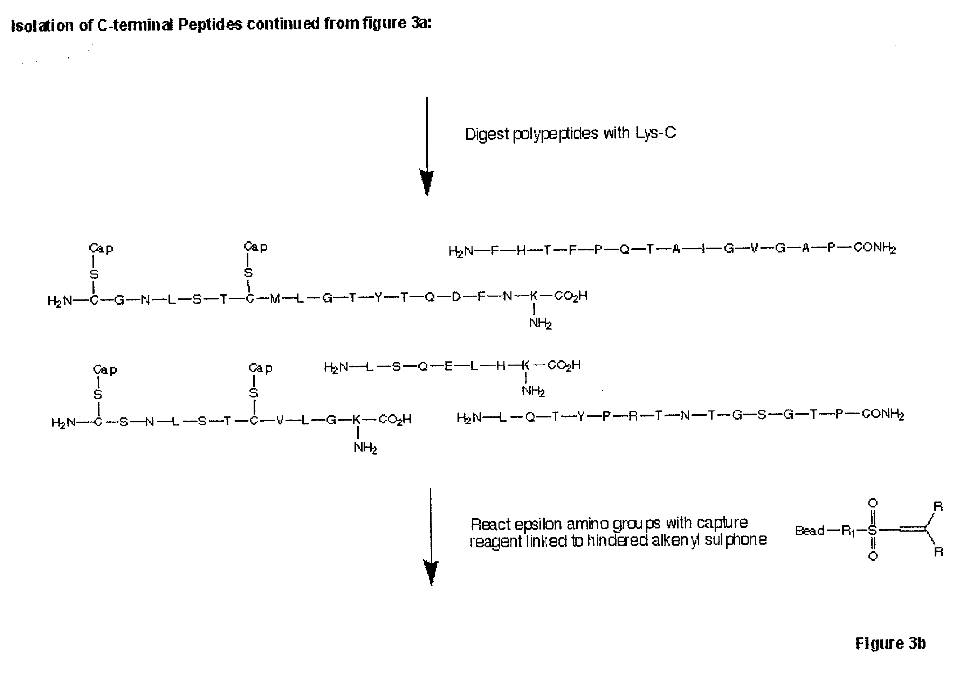

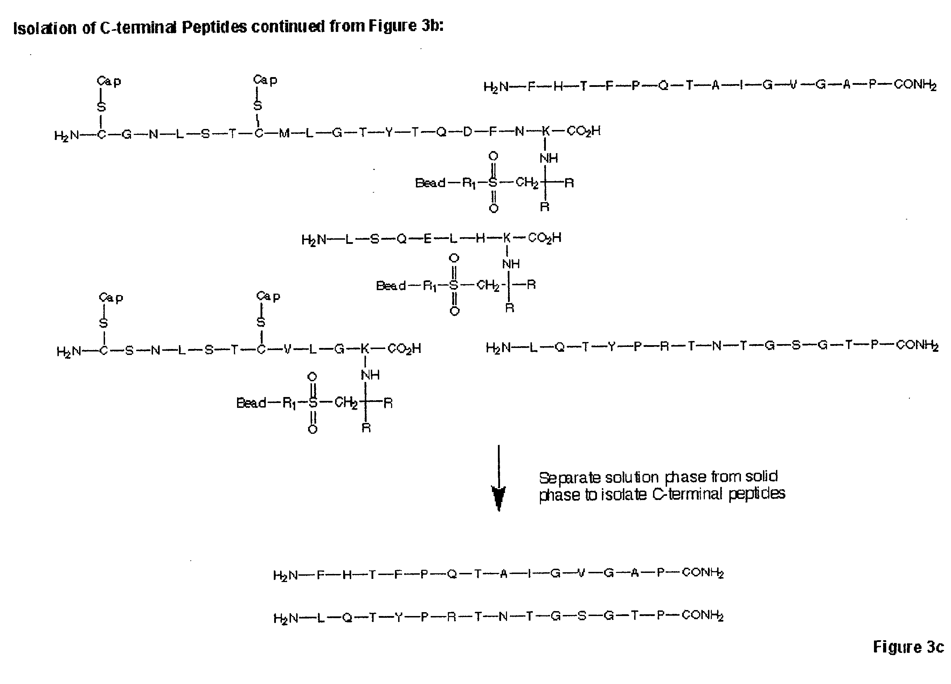

Figures 3a, 3b and 3c will now be described in more detail. Figures 3a to 3c illustrate the first embodiment of this invention, which provides a method of isolating a population of C-terminal peptides from a sample of polypeptides. Figure 3a illustrates an optional, but preferable, first step of this process in which two short polypeptides are reduced and free thiols are capped. Two polypeptides, rather than a complex mixture, are shown, Calcitonin H and Calcitonin S, for ease of illustration.

Figure 3b illustrates the second step of this embodiment of the invention in which the polypeptides are cleaved with a sequence specific cleavage reagent that cleaves at the amide bond on the C-terminal side of lysine residues. In the figure this step is performed with Lys-C. The cleavage reaction generates new free alpha-amino groups in the C-terminal product peptides of each cut.

Figure 3c illustrates the third step of this embodiment of the invention in which the epsilon amino groups in the cleaved peptides are reacted with a capture reagent. In the figure this reagent is a bead derivitised with a hindered alkenyl sulphone reagent, which reacts selectively with lysine. Since all non-C-terminal peptides have a free epsilon amino group, these peptides will react with the capture reagent. The C-terminal peptides will not be captured as they have no lysine groups. Figure 3c also illustrates the final step in this embodiment of the invention in which the C-terminal peptides are separated from non-C-terminal peptides by separating the solution phase from the beads. The C-terminal peptides remain in solution and can be analysed further. Note that cleavage with Lys-C leaves a free alpha amino group available in each C-terminal peptide. This group can be reacted with a label if desired.

The lysine reactive (lysine selective) reagents used in the methods of the present invention will now be described in more detail.

Many amine selective protein reactive reagents are known in the art. These reagents will all have some degree of discrimination in favour of reaction with lysine over alpha amino groups at high pH, but not many show sufficient discrimination to allow lysine to be labelled almost exclusively in preference to alpha amino groups. A number of lysine-selective reagents have been described in the prior art and these are all appropriate for use with this invention, particularly cyclic anhydrides. Pyromellitic dianhydride and o-sulphobenzoic acid anhydride are reported to be lysine selective acylating reagents (Bagree et al., FEBS Lett. 120 (2):275-277, 1980). Similarly Phthalic anhydride, whose structure and reactivity is similar to pyromellitic anhydride would be expected to be lysine selective. Phthalic anhydride is reported to have few side-reactions with other amino acids (Palacian E. *et al.*, Mol Cell Biochem. 97 (2): 101-111, 1990). However, many widely used reagents that react with lysine are not stable at high pH, particularly active esters such as carboxylic acid anhydrides, N-hydroxysuccinimide esters and pentafluorophenyl esters. These reagents must be used in large excess exacerbating the lack of selectivity of the reaction as a result of the excess.

Michael reagents have a number of properties that make them attractive for protein reactions and have been used quite widely for this purpose (Friedman M. & Wall J.S., J Org Chem. 31, 2888-2894, 'Additive Linear Free-Energy Relationships in Reaction Kinetics of Amino Groups with alpha-,beta-Unsaturated Compounds.' 1966; Morpurgo M. & Veronese F.M. & Kachensky D. & Harris J.M., Bioconjug. Chem. 7(3): 363-368, 'Preparation of characterization of poly(ethylene glycol) vinyl sulfone.' 1996; Friedman M. & Finley J.W., Int. J. Pept. Protein Res. 7(6): 481 - 486, 'Reactions of proteins with ethyl vinyl sulfone.' 1975; Masri M.S. & Friedman M., J Protein Chem. 7(1): 49-54, 'Protein reactions with methyl and ethyl vinyl sulfones' 1988; Graham L. & Mechanic G. L., Anal. Biochem. **153**(2): 354-358, '[14C]acrylonitrile: preparation via a stable tosylate intermediate and quantitative reaction with amine residues in collagen.' 1986; Esterbauer H. & Zollner H. & Scholz N., Z Naturforsch [C] 30 (4): 466-473, 'Reaction of glutathione with conjugated carbonyls.' 1975).

There is a number of these reagents that are relatively stable in aqueous solution and the structures of these compounds can be varied extensively to achieve different degrees of reactivity and selectivity. Other reagents used for protein labelling are often not very stable in water and are less easily modified. In particular, reactions with amino-groups in proteins are often done with active esters, which are quite susceptible to hydrolysis. Reagents based on sulphones may be more convenient and effective for labelling amino-groups than the more widely used active esters. Michael reagents that have been used with proteins include compounds such as acrylonitrile, acrylamide, vinyl pyridine, methylvinyl sulphone and methylvinyl ketone. The reactions of these compounds have been compared (Friedman M. & Wall J.S from above) and linear relationships between the reaction kinetics of these structurally similar compounds are observed. These linear relationships indicate that the reactions of this class of compounds take place by the same mechanism although their rates of reaction differ. The authors found that the sulphone and ketone compounds were by far the most reactive reagents. The vinyl compounds, i.e. acrylonitrile, acrylamide, vinyl pyridine, methylvinyl sulphone and methylvinyl ketone have broadly the same relative rates of reaction with different substrates but differ from each other in their overall rates of reaction. These linear relationships make it reasonable to assume that the reactions of this class of compounds take place by the same mechanism and that changes to substituents in this class of compounds, particularly at the beta position of the reactive double bond, will produce similar changes in behaviour in the whole class of compounds. For example, it would be expected that the change in relative reaction rates of crotononitrile with a series of substrates when compared with acrylonitrile would be essentially the same as the change in relative reaction rates of methyl propenyl sulphone with a series of substrates when compared with methyl vinyl sulphone. This means that the properties of methyl propenyl sulphone will be essentially the same as crotononitrile except that the rate of reaction of the sulphone will be faster.

The choice of a Michael reagent for the purposes of this invention is dependent on a number of criteria, included rates of reaction, chances of side-reactions apart from the Michael addition and ease of synthesis of different variants of the compound. Vinyl ketones can, for example, undergo other reactions besides Michael addition, particularly nucleophilic attack of the ketone after Michael addition has taken place. The ketone functionality can undergo this further reaction with a variety of nucleophiles, including the usual biological nucleophiles. Similarly, nitrile compounds can undergo hydrolysis of the nitrile functionality to the carboxylic acid, although typically this reaction will not occur under the conditions used in most biological assays. Alkenyl sulphones do not undergo reactions other than the Michael addition under the conditions used in typical biological assays. Alkenyl sulphones generally react rapidly with biological nucleophiles and there is an extensive literature on the synthesis of different forms of alkenyl sulphone. For these reasons alkenyl sulphones are preferred Michael Reagents for use in the biological assays of this invention. Maleimide compounds such as N-ethylmaleimide also react rapidly with proteins by Michael addition and are reasonably stable under the conditions used for labelling proteins, although alkaline hydrolysis is observed when these reagents are polymer bound. Thus maleimide compounds are also preferred Michael Reagents for use in the biological assays of this invention. In most circumstances nitrile reagents are also preferred reagents although a nitrile reagent will tend to react more slowly than corresponding sulphones. Similarly acrylamides react still more slowly. These preferences do not mean that the other Michael reagents available are unsuitable for this invention, but for most purposes rapid reaction of the reagents is preferred. Under appropriate conditions almost any of the Michael reagents could be used in the methods of this invention.

A preferred class of lysine-selective capture reagents for use in this invention comprise hindered alkenyl sulphones as the lysine selective reactive groups. Combinations of these reagents under appropriate mild conditions can allow a high degree of discrimination between alpha-amino groups and lysine epsilon-amino groups in amine-labelling reactions. Vinyl sulphones are known to react readily with primary amines giving a di-alkylated product. The inventors have shown that these reagents will react more rapidly with epsilon-amino groups at high pHs (>9.0) than with alpha-amino groups, but the discrimination of these unhindered sulphones is not especially marked. More hindered alkenyl sulphones such as propenyl sulphones and butenyl sulphones show a greatly enhanced discrimination in favour of epsilon-amino groups when compared with the vinyl sulphones. In addition, these hindered reagents produce the mono-alkylated product almost exclusively.

This discrimination by hindered sulphones means that epsilon-amino groups can be selectively labelled in preference to alpha-amino groups under mild aqueous conditions with convenient, stable, water-soluble reagents. For the purposes of this invention, a lysine selective capture reagent is required. Capture reagents may comprise the hindered alkenyl sulphone functional groups of this invention covalently linked to a solid support. Alternatively an affinity capture reagent can be generated by linking the hindered alkenyl sulphone functional groups of this invention to affinity capture functionalities such as biotin or digoxigenin. As a further alternative the hindered alkenyl sulphone functionalities may be covalently linked to a second reactive functionality that is reactive with an appropriately derivitised solid phase support. Boronic acid is known to selectively react with vicinal cis-diols and chemically similar ligands, such as salicylhydroxamic acid. Reagents comprising boronic acid have been developed for protein capture onto solid supports derivitised with salicylhydroxamic acid (Stolowitz M.L.. et al., Bioconjug Chem. 12 (2): 229-239, "Phenylboronic Acid-Salicylhydroxamic Acid Bioconjugates. 1. A Novel Boronic Acid Complex for Protein Immobilization." 2001; Wiley J.P. et al., Bioconjug. Chem. 12 (2): 240-250, "Phenylboronic Acid-Salicylhydroxamic Acid Bioconjugates. 2. Polyvalent Immobilization of Protein Ligands for Affinity Chromatography." 2001, Prolinx, Inc, Washington State, USA). It is anticipated that it should be relatively simple to link a phenylboronic acid functionality to a hindered alkenyl sulphone functionality to generate capture reagents that can be captured by selective chemical reactions. The use of this sort of chemistry would not be directly compatible with proteins bearing vicinal cis-diol-containing sugars, however these sorts of sugars could be blocked with phenylboronic acid or related reagents prior to reaction with boronic acid derivitised lysine selective reagents. Solution phase capture reagents, that may be captured onto solid supports, are advantageous as the lysine reaction may take place in the solution phase, with a large excess of reagent to drive the reaction to completion quickly.

Numerous methods of synthesising hindered alkenyl sulphones are known in the art. For general reviews of synthetic methods that have been used for the synthesis of alpha-,beta-unsaturated sulphones see Simpkins N., Tetrahedron 46, 6951-6984, 'The chemistry of vinyl sulphones', 1990; and Fuchs P. L. and Braish T. F., Chem. Rev. 86, 903-917, 'Multiply Convergent Synthesis via Conjugate-Addition Reactions to Cycloalkenyl Sulfones', 1986.

Preferred hindered alkenyl sulphone compounds of this invention have the formula:

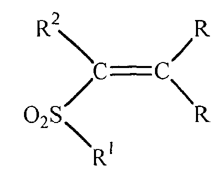

Where R¹ may be any alkyl or aromatic group but is preferably an electron withdrawing group and more preferably a cyclic or heterocylic aromatic ring or fused ring. Preferably the ring structure is electron withdrawing. More specifically R¹ is preferably a small ring or fused ring such as a phenyl, pyridyl, naphthyl or quinolyl ring structure. Preferred ring structures are substituted with appropriate electron withdrawing groups such as halogens like fluorine or nitro groups. Preferred ring structures promote water solubility, such as pyridyl and naphthyl rings. R¹ may additionally comprise a linker to an affinity capture functionality, such as biotin, or a linker to a solid phase support.

In the formula above R² is either a hydrogen atom or it may comprise an electron-withdrawing group and/or a linker to an affinity capture functionality or a linker to a solid phase support.

To be a 'hindered' Michael reagent according to this invention, at least one of the R groups is not hydrogen and is considered to be a sterically hindering group. At least one R group may comprise an alkyl or aromatic group such as a methyl or phenyl group. More preferably at least one of the R groups is electron-withdrawing and may comprise a halogen atom or a halogenated alkyl group, such as fluoromethyl, difluoromethyl or trifluoromethyl group or a phenyl ring with electron withdrawing substituents such as halogen or nitro groups. In addition, one R-group may comprise a linker to an affinity capture functionality, such as biotin, or a linker to a solid phase support. Conversely to be an 'unhindered' Michael reagent in the context of this invention, both R groups would be hydrogen.

One and preferably, only one of the R-, R¹- and R²- groups comprises a linker to an affinity capture functionality, such as biotin, or a linker to a solid phase support.

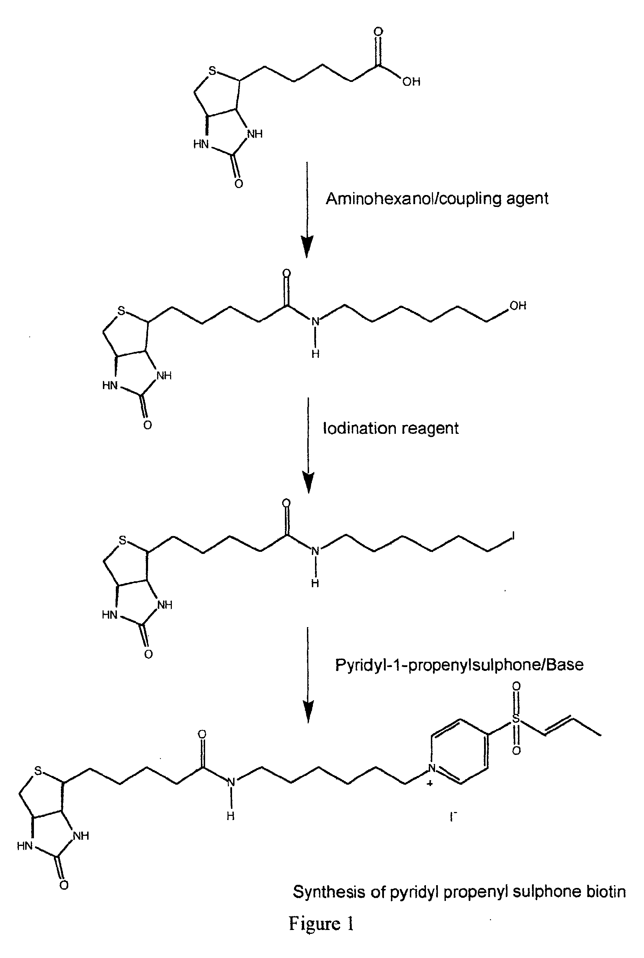

Various entry points into the synthesis of alkenyl sulphones may be contemplated to produce compounds that are appropriately substituted for use with this invention. Aldol condensation-type reactions can be used. Methyl phenyl sulphone can be reacted with a variety of ketones and aldehydes to give hindered alkenyl sulphones (see Figure 1 and the reviews above). Appropriate ketones include acetone and trifluoroacetone. Aldehydes include benzaldehyde, fluorobenzaldehyde, difluorobenzaldehyde, trifluoromethylbenzaldehyde and nitrobenzaldehyde. 4-(methylsulfonyl)benzoic acid provides a starting point for the synthesis of a hindered sulphone that can be linked to a solid support or to an affinity capture reagent through the benzoic acid. Amino-derivitised polystyrene is available from various sources including Sigma-Aldrich, UK. Carbodiimide coupling of the functionalised benzoic acid to generate an amide linkage to the solid support would be sufficient to generate a solid support derivitised with the appropriate alkenyl sulphone. Various forms of amino-functionalised biotin are available from Pierce Chemical Company, IL, USA, which would allow a biotin compound derivitised with a variety of alkenyl sulphones to be synthesised.

Synthetic routes for the production of phenyl-1-propenyl, pyridine-1-propenyl, phenyl-1-isobutenyl and pyridine-1-isobutenyl sulphones are described in the Examples below. A synthetic route for the production of 1,1,1-trifluoro-3-phenylsulphonylpropene is disclosed by Tsuge H. *et al.* in J. Chem. Soc. Perkin Trans. 1, 2761-2766, 1995. This reagent is also available from Aldrich (Sigma-Aldrich, Dorset, UK).

A second preferred class of reagents for use in this invention are maleimide compounds. Combinations of these reagents under appropriate mild conditions can allow a high degree of discrimination between alpha-amino groups and lysine epsilon-amino groups in amine-labelling reactions. Maleimide compounds are known to react readily with primary amines giving a mono-alkylated product. The inventors have shown that a solid support derivitised with maleimide (maleimidobutyramidopolystyrene, Fluka) will react more rapidly with epsilon-amino groups under basic conditions than with alpha-amino groups. This reagent is not stable in aqueous conditions, however, and reactions of peptides with this support must be carried out in anhydrous aprotic organic solvents. The use of organic solvents is acceptable for highly hydrophobic proteins, such as proteins embedded in cell membranes and as such maleimidobutyramidopolystyrene maybe useful for the analysis of this class of proteins.

Some of the less hindered Michael reagents, such as N-ethylmaleimide (NEM) and the propenyl sulphones will react quite readily with the alpha-amino group of proline. This will not be a problem in most aspects of this invention as proline is not common and most endoproteases do not cleave at proline linkages anyway. The first embodiment of this invention which provides a method to isolate C-terminal peptides, relies on cleavage of proteins and polypeptides by Lys-C type enzymes. Most of the known enzymes of this class will not cleave at Lysine-Proline linkages, so the presence of a free proline alpha-amino will not be a problem. Solid-support bound maleimide also discriminates effectively against proline. It is worth noting that maleimide shows only moderate discrimination for epsilon amino groups over alpha amino groups when used as a solution phase reagent, but the discrimination of the immobilised reagent is greatly improved. It is anticipated that other reagents, which show only moderate discrimination in the solution phase will show improved discrimination when immobilised on a solid phase support.

In the first embodiment of this invention, which describes a method to isolate all C-terminal peptides from a population of polypeptides, the discrimination of the hindered sulphones is used to capture peptides with epsilon-amino groups. In the first step of this embodiment a sample of polypeptides is cleaved with a sequence specific cleavage reagent peptide at the amide bond C-terminal to a lysine residue, such as Lys-C. The cleavage of the mixture of polypeptides will produce a mixture of peptides with lysine epsilon-amino groups in all but the C-terminal peptides. These epsilon-amino groups can be reacted with a hindered sulphone reagent of the invention, which is either linked to a capture reagent, such as biotin, or it is linked to a solid support. This step allows all non-C-terminal peptides to be captured leaving the C-terminal peptides free in solution. These C-terminal peptides have a free alpha amino functionality that may then be labelled further and may be analysed by any appropriate technique, particularly mass spectrometry.

A further aspect of this invention, provides a method of determining the 'expression profile' of a mixture of polypeptides, i.e. a method to identify and preferably also to quantify each polypeptide in the mixture. These methods involve isolating peptides according to the first embodiment of the invention, optionally labelling the peptides with a mass marker and analysing the peptides by mass spectrometry. Preferred labels for use with this invention are disclosed in PCT/GB01/01122, which discloses organic molecule mass markers that are analysed by selected reaction monitoring. This application discloses two component mass markers connected by a collision cleavable group. Sets of tags are synthesised where the sum of the masses of the two components produces markers with the same overall mass. The mass markers may be analysed after cleavage from their analyte or may be detected while attached to the analyte. In this invention the mass markers are detected while attached to the peptide that they are identifying. Selection of the mass of the mass marker with its associated peptide by the first mass analyser of a tandem instrument allows the marked peptides to be abstracted from the background. Collision of the markers in the second stage of the instrument separates the two components of the tag from each other. Only one of these components is detected in the third mass analyser. This allows confirmation that the peak selected in the first analyser is a mass marked peptide. The whole process greatly enhances the signal to noise ratio of the analysis and improves sensitivity. This mass marker design also compresses the mass range over which an array of mass markers is spread. Moreover, it allows the design of markers, which are chemically identical, have the same mass but which are still resolvable by mass spectrometry. This is essential for analytical techniques such as Liquid Chromatography Mass Spectrometry (LC-MS) where the effect of different markers on the mobility of different samples of peptides must be minimised so that corresponding peptides from each sample elute together into the mass spectrometer, allowing the ratios of the corresponding peptides to be determined. These markers are thus most preferred for the purposes of this invention because of the use of high selectivity detection and the closely related structures of these markers. Other markers may also be applicable, though.

The reagents of this invention are reactive with free thiols. To prevent interference in the methods of this invention by free thiols and to avoid problems associated with disulphide bridges in polypeptides, it is preferred that the disulphide bridges are reduced to free thiols and that the thiol moieties are capped prior to reaction of lysine residues with lysine selective capture reagents. Since thiols are very much more reactive than the other side-chains in a protein this step can be achieved highly selectively. Discrimination between thiols and epsilon amino groups may be achieved quite effectively by control of pH. At pH thiol reactions take place almost exclusively, while reactions of epsilon amines require a pH of 9 or greater for any reaction to take place at a meaningful rate.

Various reducing agents have been used for disulphide bond reduction. The choice of reagent may be determined on the basis of cost, ease of use or efficiency of reaction and compatibility with the reagents used for capping the thiols (for a review on these reagents and their use see Jocelyn P.C., Methods Enzymol. 143, 246-256, 'Chemical reduction of disulfides.' 1987).

Typical capping reagents include N-ethylmaleimide, iodoacetamide, vinylpyridine, 4-nitrostyrene, methyl vinyl sulphone or ethyl vinyl sulphone (see for example Krull L. H. & Gibbs D. E. & Friedman M., Anal. Biochem. 40 (1): 80-85, '2-Vinylquinoline, a reagent to determine protein sulfhydryl groups spectrophotometrically.' 1971; Masri M. S. & Windle J. J. & Friedman M., Biochem Biophys. Res. Commun. 47 (6): 1408-1413, 'p-Nitrostyrene: new alkylating agent for sulfhydryl groups in reduced soluble proteins and keratins.' 1972; Friedman M. & Zahnley J.C. & Wagner J.R., Anal. Biochem. 106 (1): 27-34, 'Estimation of the disulfide content of trypsin inhibitors as S-beta-(2-pyridylethyl)-L-cysteine." 1980).

Typical reducing agents include mercaptoethanol, dithiothreitol (DTT), sodium borohydride and phosphines such as tributylphosphine (see Ruegg U. T. & Rudinger J., Methods Enzymol. 47, 111-116, 'Reductive cleavage of cysteine disulfides with tributylphosphine.', 1977) and tris(carboxyethyl)phosphine (Burns J.A. et al., J Org Chem. 56, 2648-2650, 'Selective reduction of disulfides by tris(2-carboxyethyl)phosphine.', 1991). Mercaptoethanol and DTT may be less preferred for use with thiol reactive capping reagents as these compounds contain thiols themselves. Phosphine based reducing reagents are compatible with vinyl sulphone reagents (Masri M. S. & Friedman M., J. Protein Chem. 7 (1): 49-54, 'Protein reactions with methyl and ethyl vinyl sulfones.' 1988).

In the first embodiment of this invention a population of polypeptides is completely digested with a cleavage reagent that cuts a polypeptide or peptide at the amide bond C-terminal to a lysine residue. Various enzymes with this property are commercially available, e.g. Endoproteinase Lys-C from Lysobacter enzymogenes (Formerly available from Boehringer Mannheim now from Roche Biochemicals).

### Fractionating Proteins and Peptides

The methods of this invention can be used to profile populations of proteins generated in numerous ways. It may be possible to analyse raw protein extracts from organisms such as yeast directly using the methods of this invention. Organisms with larger proteomes may require fractionation of the raw protein extracts from their tissues. Various fractionation techniques exist to sub-sort proteins on the basis of certain features. A population of proteins extracted from a mammalian tissue, for example, is going to contain a significant number of distinct protein species. It is thought there are of the order of 10,000 transcripts, which may comprise alternatively spliced products from numerous genes, expressed in the average human cell (Iyer V.R. et al., Science 283 (5398) 83-87, "The transcriptional program in the response of human fibroblasts to serum." 1999), and experiments with 2-D gels have shown that similar numbers of proteins spots are found in gels of proteins extracted from a particular tissue (Klose J., Kobalz U., Electrophoresis 16 (6) 1034-59, "Two-dimensional electrophoresis of proteins: an updated protocol and implications for a functional analysis of the genome." 1995). It may be desirable to fractionate complex samples of proteins, such as those that would be isolated from human tissue, prior to application of the methods of this invention to simplify analysis or to provide additional information, such as identifying proteins with post-translational modifications. It may also be desirable to fractionate the terminal peptides isolated from a population of proteins using the methods of this invention prior to further manipulations or analysis.

Fractionation steps can be used to reduce the complexity of a population of proteins by resolving a protein population into a number of discrete subsets, preferably subsets of a uniform size are desirable. This is most readily achieved by separation on the basis of global properties of proteins, that vary over a broad and continuous range, such as size and surface charge. These are the properties used most effectively in 2-D gel electrophoresis. Such separations can be achieved more rapidly than gel electrophoresis using liquid chromatographic techniques. By following one liquid chromatography separation by another, a population of proteins can be resolved to an arbitrary degree, although a large number of sequential chromatographic separation steps could result in sample loss or other artefacts due to non-specific adhesion of proteins or peptides to different chromatographic matrices.

### Cell fractionation

Proteins are compartmentalised within their cells. Various techniques are known in the art to fractionate proteins on the basis of their cellular compartments. Fractionation protocols involve various cell lysis techniques such as sonication, detergents or mechanical cell lysis that can be followed by a variety of fractionation techniques, such as centrifugation. Separation into membrane proteins, cytosolic proteins and the major membrane bound subcellular compartments, such as the nucleus and mitochondria, is standard practice. Thus certain classes of protein may be effectively ignored or can be specifically analysed. This form of fractionation may be extremely informative if a particular protein is found in a number of subcellular locations since its location is likely to reveal information about its function.

### Fractionation of proteins and peptides

Since proteins are highly heterogeneous molecules numerous techniques for separation of proteins are available. It is possible to separate proteins on the basis of size, hydrophobicity, surface charge and/or by affinity to particular ligands. Separation is effected by an assortment of solid phase matrices derivatised with various functionalities that adhere to and hence slow down the flow of proteins through the column on the basis of specific properties. Matrices derivitised with hydrophobic moieties can be used to separate proteins based on their hydrophobicity, while charged resins can be used to separate proteins on the basis of their charge. In a typical chromatographic separation, analyte molecules are injected into columns packed with these a derivitised resin in a loading buffer or solvent that favours adhesion to the solid phase matrix. This is followed by washing the column with steadily increasing quantities of a second buffer or solvent favouring elution. In this way the proteins with the weakest interactions with a given matrix elute first.

It is desirable, after isolation of terminal peptides using the methods of this invention, to analyse the resultant peptides. Fractionation of the terminal peptides generated by the methods of this invention is optional but in populations comprising large numbers of peptides, detection and identification of peptides is greatly facilitated by analytical separation steps. Various liquid chromatography techniques have been used for peptide separations. A preferred technique is High Pressure Liquid Chromatography (HPLC) as this technique combines rapid separation of small volumes of analyte solution whilst also achieving very good resolution of peptides. In HPLC the matrix is designed to be highly incompressible allowing chromatographic separation to be performed at extremely high pressures, which favours rapid and discrete separation. These features make HPLC very attractive for use with mass spectrometry, which is a preferred detection technology for use with peptides. Liquid chromatography mass spectrometry (LCMS) is a well developed field. HPLC systems in-line with electrospray mass spectrometers are in widespread use. HPLC is a fast and effective way of resolving peptide samples generated by the methods of this invention.

Other fractionation procedure may be used as part of the analysis of a population of terminal peptides prior to mass spectrometry depending on the configuration of the mass spectrometer used. Sorting peptides by ion exchange chromatography, for example, may be advantageous, in that short peptides could be separated in an almost sequence dependent manner: the amino acids that are ionisable have known pKa values and hence elution of peptides from such a column at a specific pH, would be indicative of the presence of particular amino acids in that sequence. For example, aspartate residues have a pKa of 3.9 and glutamate residues 4.3. Elution of a peptide at pH 4.3 would be indicative of the presence of glutamate in the peptide. These effects are sometimes masked in large proteins but should be more distinct in short peptides. Fractions could be analysed by spotting onto a target for subsequent analysis by laser desorption analysis (discussed later in the text). Alternatively an 'autosampler' can be used to inject fractions from chromatographic separations into an electrospray ionisation mass spectrometer system.

### Fractionation by Affinity

A population of proteins can be fractionated by affinity methods. This sort of fractionation method relies on specific interactions between proteins, or classes of proteins, with specific ligands.

Many proteins, for example, exist as complexes with other proteins and analysis of such complexes is often difficult. A cloned protein that is a putative member of a complex can be used to generate an affinity column with the cloned protein acting as an affinity ligand to capture other proteins that normally bind to it. This invention is eminently suited to the analysis of such captured protein complexes.

### Isolation of post-translationally modified proteins

A large number of affinity ligands are available commercially for specific applications such as the isolation of proteins with post-translational modifications. A number of tagging procedures are also known by which affinity tags such as biotin can be introduced into proteins that have specific post-translational modifications allowing such proteins to be captured using biotin-avidin affinity chromatography.

### Isolation of carbohydrate modified proteins

Carbohydrates are often present as a post-translational modification of proteins. Various affinity chromatography techniques for the isolation of these sorts of proteins are known (For a review see Gerard C., Methods Enzymol 182, 529-539, "Purification of glycoproteins." 1990). A variety of natural protein receptors for carbohydrates are known. The members of this class of receptors, known as lectins, are highly selective for particular carbohydrate functionalities. Affinity columns derivitised with specific lectins can be used to isolate proteins with particular carbohydrate modifications, whilst affinity columns comprising a variety of different lectins could be used to isolate populations of proteins with a variety of different carbohydrate modifications. Many carbohydrates have vicinal-diol groups present, i.e. hydroxyl groups present on adjacent carbon atoms. Diol containing carbohydrates that contain vicinal diols in a 1,2-cis-diol configuration will react with boronic acid derivatives to form cyclic esters. This reaction is favoured at basic pH but is easily reversed at acid pH. Resin immobilised derivatives of phenyl boronic acid have been used as ligands for affinity capture of proteins with cis-diol containing carbohydrates. Vicinal-diols, in sialic acids for example, can also be converted into carbonyl groups by oxidative cleavage with periodate. Enzymatic oxidation of sugars containing terminal galactose or galactosamine with galactose oxidase can also convert hydroxyl groups in these sugars to carbonyl groups. Complex carbohydrates can also be treated with carbohydrate cleavage enzymes, such as neuramidase, which selectively remove specific sugar modifications leaving behind sugars, which can be oxidised. These carbonyl groups can be tagged allowing proteins bearing such modifications to be detected or isolated. Biocytin hydrazide (Pierce & Warriner Ltd, Chester, UK) will react with carbonyl groups in carbonyl-containing carbohydrate species (E.A. Bayer *et al.*, Anal. Biochem. 170, 271-281, "Biocytin hydrazide - a selective label for sialic acids, galactose, and other sugars in glycoconjugates using avidin biotin technology", 1988). Alternatively a carbonyl group can be tagged with an amine modified biotin, such as Biocytin and EZ-Link™ PEO-Biotin (Pierce & Warriner Ltd, Chester, UK), using reductive alkylation (Means G.E., Methods Enzymol 47, 469-478, "Reductive alkylation of amino groups." 1977; Rayment I., Methods Enzymol 276: 171-179, "Reductive alkylation of lysine residues to alter crystallization properties of proteins." 1997). Proteins bearing vicinal-diol containing carbohydrate modifications in a complex mixture can thus be biotinylated. Biotinylated, hence carbohydrate modified, proteins may then be isolated using an avidinated solid support.

Terminal peptides may then be isolated from the captured carbohydrate bearing proteins isolated using the above methods and others known in the art.

### Isolation of phosphorylated proteins

Phosphorylation is a ubiquitous reversible post-translational modification that appears in the majority of signalling pathways of almost all organisms. It is an important area of research and tools which allow the analysis of the dynamics of phosphorylation are essential to a full understanding of how cells responds to stimuli, which includes the responses of cells to drugs.

A number of research groups have reported on the production of antibodies, which bind to phosphotyrosine residues in a wide variety of proteins. (see for example A.R. Frackelton et al., Methods Enzymol 201, 79-92, "Generation of monoclonal antibodies against phosphotyrosine and their use for affinity purification of phosphotyrosine-containing proteins.", 1991 and other articles in this issue of Methods Enzymol.). This means that a significant proportion of proteins that have been post-translationally modified by tyrosine phosphorylation may be isolated by affinity chromatography using these antibodies as the affinity column ligand.

These phosphotyrosine binding antibodies can be used in the context of this invention to isolate terminal peptides from proteins containing phosphotyrosine residues. The tyrosine-phosphorylated proteins in a complex mixture may be isolated using anti-phosphotyrosine antibody affinity columns. The C-terminal peptides from the fractionated mixture of phosphoproteins may then be isolated according to the methods of this invention.

Techniques for the analysis of phosphoserine and phosphothreonine containing peptides are also known. One class of such methods is based a well known reaction for beta-elimination of phosphates. This reaction results in phosphoserine and phosphothreonine forming dehydroalanine and methyldehydroalanine, both of which are Michael acceptors and will react with thiols. This has been used to introduce hydrophobic groups for affinity chromatography (See for example Holmes C.F., FEBS Lett 215 (1) 21-24, "A new method for the selective isolation of phosphoserine-containing peptides." 1987). Dithiol linkers have also been used to introduce fluorescein and biotin into phosphoserine and phosphothreonine containing peptides (Fadden P, Haystead TA, Anal Biochem 225 (1) 81-8, "Quantitative and selective fluorophore labelling of phosphoserine on peptides and proteins: characterization at the attomole level by capillary electrophoresis and laser-induced fluorescence." 1995; Yoshida O. *et al.*, Nature Biotech 19, 379-382, "Enrichment analysis of phosphorylated proteins as a tool for probing the phosphoproteome", 2001). The use of biotin for affinity enrichment of proteins phosphorylated at serine and threonine could be used with the methods of this invention so that only the terminal peptides need to be analysed. Similarly anti-fluorescein antibodies are known which would allow fluorescein tagged peptides to be selectively isolated with affinity chromatography. This could be followed by terminal peptide isolation according to the methods of this invention.

A chemical procedure for the isolation of phosphoproteins onto solid phase supports has also been published (Zhou H et al., Nature Biotech 19, 375-378, "A systematic approach to the analysis of protein phosphorylation", 2001). This procedure relies on the fact that phosphoramidates hydrolyse easily under acid conditions. The procedure involves capping all free amines in a mixture of proteins, followed by blocking all free phosphates and carboxyl groups by coupling the phosphates and carboxyls with a capping group containing an amine functionality to form the corresponding phosphoramidates and amides. The blocked proteins are then treated with acid to unblock the phosphates. The peptides are then reacted with a second amine reagent carrying a protected thiol. This step blocks the phosphates again. The protected thiol was deprotected and used to capture the phosphopeptides selectively onto a thiol reactive resin. These peptides could then be released by acid hydrolysis, after thorough washing of the resin. This procedure is claimed to be applicable to all phosphate groups but phosphotyrosine is acid labile and so the method is unlikely to applicable to phosphotyrosine.

Immobilised Metal Affinity Chromatography (IMAC) represents a further technique for the isolation of phosphoproteins and phosphopeptides. Phosphates adhere to resins comprising trivalent metal ions particularly to Gallium(III) ions (Posewitch, M.C. and Tempst, P., Anal. Chem., 71: 2883-2892, "Immobilized Gallium (III) Affinity Chromatography of Phosphopeptides", 1999). This technique is advantageous as it can isolate both serine/threonine phosphorylated and tyrosine phosphorylated peptides and proteins simultaneously.

IMAC can therefore also be used in the context of this invention for the analysis of samples of phosphorylated proteins. In an alternative embodiment of the second aspect of this invention, a sample of phosphorylated proteins may be analysed by isolating phosphorylated proteins followed by analysis of the C terminal peptides of the phosphoproteins. A protocol for the analysis of a sample of proteins, which contains phosphorylated proteins, would comprise the steps of:
1. passing the protein sample through an affinity column comprising immobilised metal ions to isolate only phosphorylated proteins,
2. isolating C peptides from the captured phosphorylated proteins using the methods of this invention,
3. analysing the tagged peptides by LC-MS-MS.

### Other post-translational modifications of proteins

Proteins that have been modified by ubiquitination, lipoylation and other post-translational modifications may also be isolated or enriched by chromatographic techniques (Gibson J.C., Rubinstein A., Ginsberg H.N. & Brown W.V., Methods Enzymol 129, 186-198, "Isolation of apolipoprotein E-containing lipoproteins by immunoaffinity chromatography." 1986; Tadey T. & Purdy W.C. J Chromatogr. B Biomed. Appl. 671 (1-2), 237-253, "Chromatographic techniques for the isolation and purification of lipoproteins." 1995) or affinity ligand based techniques such as immunoprecipitation (Hershko A., Eytan E., Ciechanover A. & Haas A.L., J. Biol. Chem. 257, (23) 13964-13970, "Immunochemical analysis of the turnover of ubiquitin-protein conjugates in intact cells. Relationship to the breakdown of abnormal proteins." 1982). Populations of proteins with these modifications can all be analysed by the methods of this invention.

### The analysis of peptides using mass spectrometry

The essential features of a mass spectrometer are as follows:
Inlet System - Ion Source - Mass Analyser - Ion Detector - Data Capture System

There are certain preferred inlet systems, ion sources and mass analysers for the purposes of analysing peptides.

### Inlet systems

In all of the aspects of this invention a chromatographic or electrophoretic separation may be used to reduce the complexity of the sample prior to analysis by mass spectrometry. A variety of mass spectrometry techniques are compatible with separation technologies particularly capillary zone electrophoresis and High Performance Liquid Chromatography (HPLC). The choice of ionisation source may be limited to some extent if a separation is required as ionisation techniques such as MALDI and FAB (discussed below), which ablate material from a solid surface are less suited to chromatographic separations. It is difficult to link a chromatographic separation in-line with mass spectrometric analysis by one of these techniques. Dynamic FAB and ionisation techniques based on spraying such as electrospray, thermospray and APCI are all compatible with in-line chromatographic separations.

### Ionisation techniques

For many biological mass spectrometry applications so called 'soft' ionisation techniques are used. These allow large molecules such as proteins and nucleic acids to be ionised essentially intact. The liquid phase techniques allow large biomolecules to enter the mass spectrometer in solutions with mild pH and at low concentrations. A number of techniques are appropriate for use with this invention including but not limited to Electrospray Ionisation Mass Spectrometry (ESI-MS), Fast Atom Bombardment (FAB), Matrix Assisted Laser Desorption Ionisation Mass Spectrometry (MALDI MS) and Atmospheric Pressure Chemical Ionisation Mass Spectrometry (APCI-MS).

### Electrospray Ionisation

Electrospray ionisation requires that the dilute solution of the analyte molecule is 'atomised' into the spectrometer, i.e. injected as a fine spray. The solution is, for example, sprayed from the tip of a charged needle in a stream of dry nitrogen and an electrostatic field. The mechanism of ionisation is not fully understood but is thought to work broadly as follows. In a stream of nitrogen the solvent is evaporated. With a small droplet, this results in concentration of the analyte molecule. Given that most biomolecules have a net charge this increases the electrostatic repulsion of the dissolved molecule. As evaporation continues this repulsion ultimately becomes greater than the surface tension of the droplet and the droplet disintegrates into smaller droplets. This process is sometimes referred to as a 'Coulombic explosion'. The electrostatic field helps to further overcome the surface tension of the droplets and assists in the spraying process. The evaporation continues from the smaller droplets which, in turn, explode iteratively until essentially the biomolecules are in the vapour phase, as is all the solvent. This technique is of particular importance in the use of mass labels in that the technique imparts a relatively small amount of energy to ions in the ionisation process and the energy distribution within a population tends to fall in a narrower range when compared with other techniques. The ions are accelerated out of the ionisation chamber by the use of electric fields that are set up by appropriately positioned electrodes. The polarity of the fields may be altered to extract either negative or positive ions. The potential difference between these electrodes determines whether positive or negative ions pass into the mass analyser and also the kinetic energy with which these ions enter the mass spectrometer. This is of significance when considering fragmentation of ions in the mass spectrometer. The more energy imparted to a population of ions the more likely it is that fragmentation will occur through collision of analyte molecules with the bath gas present in the source. By adjusting the electric field used to accelerate ions from the ionisation chamber it is possible to control the fragmentation of ions. This is advantageous when fragmentation of ions is to be used as a means of removing tags from a labelled biomolecule.

### Matrix Assisted Laser Desorption Ionisation (MALDI)

MALDI requires that the biomolecule solution be embedded in a large molar excess of a photo-excitable 'matrix'. The application of laser light of the appropriate frequency results in the excitation of the matrix which in turn leads to rapid evaporation of the matrix along with its entrapped biomolecule. Proton transfer from the acidic matrix to the biomolecule gives rise to protonated forms of the biomolecule which can be detected by positive ion mass spectrometry. This technique imparts a significant quantity of translational energy to ions, but tends not to induce excessive fragmentation despite this. Accelerating voltages can again be used to control fragmentation with this technique though.

### Fast Atom Bombardment

Fast Atom Bombardment (FAB) has come to describe a number of techniques for vaporising and ionising relatively involatile molecules. In these techniques a sample is desorbed from a surface by collision of the sample with a high energy beam of xenon atoms or caesium ions. The sample is coated onto a surface with a simple matrix, typically a non volatile material, e.g. *m*-nitrobenzyl alcohol (NBA) or glycerol. These techniques are also compatible with liquid phase inlet systems - the liquid eluting from a capillary electrophoresis inlet or a high pressure liquid chromatography system pass through a frit, essentially coating the surface of the frit with analyte solution which can be ionised from the frit surface by atom bombardment.

### Mass Analysers

In most cases mass determination of each peptide will be sufficient to identify the protein from which the peptide was derived. Mass determination can be performed quite economically by using one of a number of simple mass analyser geometries such as Time Of Flight, Quadrupole and Ion Trap instruments. Fragmentation of peptides by collision induced dissociation can be used to identify proteins whose identity is not determined by the mass of its terminal peptides alone. More complex mass analyser geometries may be necessary if more information about a peptide is required, although ion traps may be sufficient for this purpose as well.

### MS/MS and MSⁿ analysis of peptides

Tandem mass spectrometers allow ions with a pre-determined mass-to-charge ratio to be selected and fragmented by collision induced dissociation (CID). The fragments can then be detected providing structural information about the selected ion. When peptides are analysed by CID in a tandem mass spectrometer, characteristic cleavage patterns are observed, which allow the sequence of the peptide to be determined. Natural peptides typically fragment randomly at the amide bonds of the peptide backbone to give series of ions that are characteristic of the peptide. CID fragment series are denoted aₙ, bₙ, cₙ, etc. for cleavage at the n^{th} peptide bond where the charge of the ion is retained on the N-terminal fragment of the ion. Similarly, fragment series are denoted xₙ, yₙ, zₙ, etc. where the charge is retained on the C-terminal fragment of the ion.

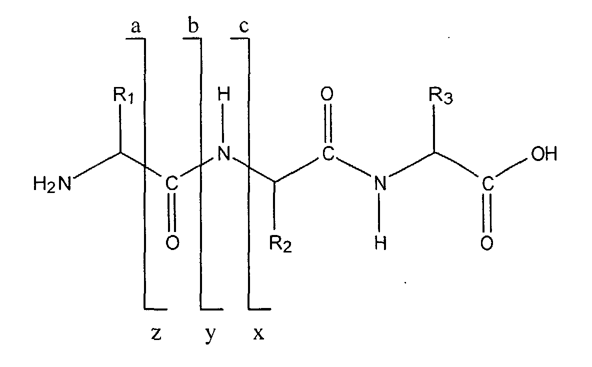

Trypsin and thrombin are favoured cleavage agents for tandem mass spectrometry as they produce peptides with basic groups at both ends of the molecule, i.e. the alpha-amino group at the N-terminus and lysine or arginine side-chains at the C-terminus. This favours the formation of doubly charged ions, in which the charged centres are at opposite termini of the molecule. These doubly charged ions produce both C-terminal and N-terminal ion series after CID. This assists in determining the sequence of the peptide. Generally speaking only one or two of the possible ion series are observed in the CID spectra of a given peptide. In low-energy collisions typical of quadrupole based instruments the b-series of N-terminal fragments or the y-series of C-terminal fragments predominate. If doubly charged ions are analysed then both series are often detected. In general, the y-series ions predominate over the b-series.

A typical tandem mass spectrometer geometry is a triple quadrupole which comprises two quadrupole mass analysers separated by a collision chamber, also a quadrupole. This collision quadrupole acts as an ion guide between the two mass analyser quadrupoles into which a gas can be introduced to allow collision with the ion stream from the first mass analyser. The first mass analyser selects ions on the basis of their mass/charge ration which pass through the collision cell where they fragment. The degree of fragmentation may be controlled by varying either the electric fields used to accelerate the ions or by varying the gas in the collision cell, e.g. helium can be replaced by neon. The fragment ions are separated and detected in the third quadrupole. Induced cleavage can be performed in geometries other than tandem analysers. Ion traps mass spectrometers can promote fragmentation through introduction of a gas into the trap itself with which trapped ions can collide after acceleration. Ion traps generally contain a bath gas, such as helium but addition of neon for example, promotes fragmentation. Similarly photon induced fragmentation could be applied to trapped ions. Another favourable geometry is a Quadrupole/Orthogonal Time of Flight tandem instrument where the high scanning rate of a quadrupole is coupled to the greater sensitivity of a reflectron TOF mass analyser to identify the products of fragmentation.

Conventional 'sector' instruments are another common geometry used in tandem mass spectrometry. A sector mass analyser comprises two separate 'sectors', an electric sector which focuses an ion beam leaving a source into a stream of ions with the same kinetic energy using electric fields. The magnetic sector separates the ions on the basis of their mass to generate a spectrum at a detector. For tandem mass spectrometry a two sector mass analyser of this kind can be used where the electric sector provide the first mass analyser stage, the magnetic sector provides the second mass analyser, with a collision cell placed between the two sectors. This geometry might be quite effective for cleaving labels from a mass labelled nucleic acid. Two complete sector mass analysers separated by a collision cell can also be used for analysis of mass labelled nucleic acids.

### Ion Traps

Ion Trap mass spectrometers are a relative of the quadrupole spectrometer. The ion trap generally has a 3 electrode construction - a cylindrical electrode with 'cap' electrodes at each end forming a cavity. A sinusoidal radio frequency potential is applied to the cylindrical electrode while the cap electrodes are biased with DC or AC potentials. Ions injected into the cavity are constrained to a stable trajectory within the trap by the oscillating electric field of the cylindrical electrode. However, for a given amplitude of the oscillating potential, certain ions will have an unstable trajectory and will be ejected from the trap. A sample of ions injected into the trap can be sequentially ejected from the trap according to their mass/charge ratio by altering the oscillating radio frequency potential. The ejected ions can then be detected allowing a mass spectrum to be produced.

Ion traps are generally operated with a small quantity of a 'bath gas', such as helium, present in the ion trap cavity. This increases both the resolution and the sensitivity of the device as the ions entering the trap are essentially cooled to the ambient temperature of the bath gas through collision with the bath gas. Collisions both increase ionisation when a sample is introduced into the trap and dampen the amplitude and velocity of ion trajectories keeping them nearer the centre of the trap. This means that when the oscillating potential is changed, ions whose trajectories become unstable gain energy more rapidly, relative to the damped circulating ions and exit the trap in a tighter bunch giving a narrower larger peaks.

Ion traps can mimic tandem mass spectrometer geometries, in fact they can mimic multiple mass spectrometer geometries allowing complex analyses of trapped ions. A single species of selected mass-to-charge ratio from a sample can be retained in a trap, i.e. all other species can be ejected. The retained species can be excited by super-imposing a second oscillating frequency on the first. The excited ions will then collide with the bath gas and will fragment if sufficiently excited. The resultant fragments can then be analysed further. It is possible to retain a fragment ion for further analysis by ejecting unwanted ions from the trap. The retained fragment may be excited again to induce further fragmentation. This process can be repeated for as long as sufficient sample exists to permit further analysis. It should be noted that these instruments generally retain a high proportion of fragment ions after induced fragmentation. These instruments and FTICR mass spectrometers (discussed below) represent a form of temporally resolved tandem mass spectrometry rather than spatially resolved tandem mass spectrometry which is found in linear mass spectrometers.

### Fourier Transform Ion Cyclotron Resonance Mass Spectrometry (FTICR MS)

FTICR mass spectrometers have similar features to ion traps in that a sample of ions is retained within a cavity but in FTICR MS the ions are trapped in a high vacuum chamber by crossed electric and magnetic fields. The electric field is generated by a pair of plate electrodes that form two sides of a box. The box is contained in the field of a magnet, which in conjunction with the two electric field-generating plates, referred to as the trapping plates, constrain injected ions to a stable cycloidal trajectory between the trapping plates, perpendicular to the applied magnetic field. The ions are excited into wider orbits by applying a radio-frequency pulse to two 'transmitter plates' which form two further opposing sides of the box. The cycloidal motions of the ions generate corresponding electric fields in the remaining two opposing sides of the box which comprise the 'receiver plates'. The excitation pulses excite ions to larger orbits which decay as the coherent motions of the ions is lost through collisions. The corresponding signals detected by the receiver plates are converted to a mass spectrum by Fourier transform analysis.

For induced fragmentation experiments these instruments can perform in a similar manner to an ion trap - all ions except a single species of interest can be ejected from the trap. A collision gas can be introduced into the trap and fragmentation can be induced. The fragment ions can be subsequently analysed. Generally fragmentation products and bath gas combine to give poor resolution if analysed by FT of signals detected by the 'receiver plates', however the fragment ions can be ejected from the cavity and analysed in a tandem configuration with a quadrupole, for example.

### Examples

### Example 1 - Isolation of C terminal peptides by capture of non-C-terminal peptides onto a solid support

An aspect of this invention provides a method of isolating C-terminal peptides from a mixture of proteins following protease digestion with either trypsin or Lys-C. After digestion the resulting mixture of peptides will contain α-amino and ε-amino groups all apart from the C-terminal peptide that will only have an α-amino group. Therefore, any compound that can preferentially react with ε-amino groups and be used to isolate these peptides away from the C-terminal peptide. Since the earlier examples in which peptides were labelled with 'free' maleimide showed that maleimide reacted reliably with epsilon amino groups and with some selectivity against alpha-amino groups and since polystyrene immobilised maleimide is commercially available (Fluka, Gillingham, Dorset, UK), its suitability as a reagent for C terminal peptide isolation has been investigated.

### Reagents

4-(maleimidobutyramidomethyl)-polystyrene beads were obtained from Fluka. These beads have a capacity of approximately 0.4mmol maleimide/g of bead.

The following pair of peptides were chosen as model peptides:
a: pro-phe-gly-lys - has α-amino and ε-amino groups
c: val-gly-ser-glu - has just an α-amino group and corresponds to the C terminal peptide

Proof of principle of this C-terminal peptide isolation protocol requires that the 'a' peptide should bind to the beads leaving the 'c' peptide (the C terminal one) in solution.

### Initial Experiment

The following experiment was performed to determine the selectivity of the reaction of immobilised maleimide with alpha amino groups. The conditions used were as follows:
400nmol of each peptide (a and b) were mixed separately with bead equivalent of either 4000 or 12000 nmol maleimide.

The reactions were shaken at RT in 12.5 mM sodium borate pH 9.5 with 25 % acetonitrile.

10µl samples removed as 0, 2, 4 hours and overnight intervals.

Following this the peptides were separated by TLC (methanol : acetic acid : ethyl acetate 1:1:2) and ninhydrin stained to assess the amount of peptide present.

### Results

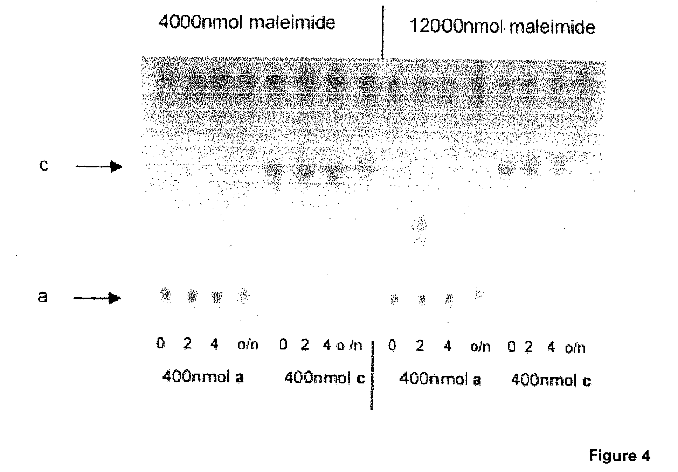

As can be seen from the TLC plate shown in figure 4, the levels of the a or c peptide do not change much over time. However, there is a slight decrease in the amount of both peptides and this could possibly result from non-specific binding of the peptides to the beads. These results suggests that the 'a' peptide is not reacting with the beads under these conditions.

It is known the maleimide hydrolyses under alkaline aqueous conditions and it is possible that the polymer-bound maleimide is hydrolysed faster than it is reacting with the 'a' peptide in the present conditions. Therefore, to address this issue, the reaction was repeated under non-aqueous conditions with an aprotic organic solvent.

### Non-aqueous reaction

To mimic a protease digest peptides a and c were mixed in equal amounts and reacted in the following way:
400 nmol of each peptide (a and b) were mixed together with bead equivalent of either 4000 or 12000 nmol maleimide all dissolved in DMF with 10 % triethylamine.

The reactions were shaken at RT and samples removed as 0 and 2 hours and overnight.

Following this the peptides were separated by TLC (methanol : acetic acid : ethyl acetate 1:1:2) and ninhydrin stained to assess the amount of peptide present.

### Results

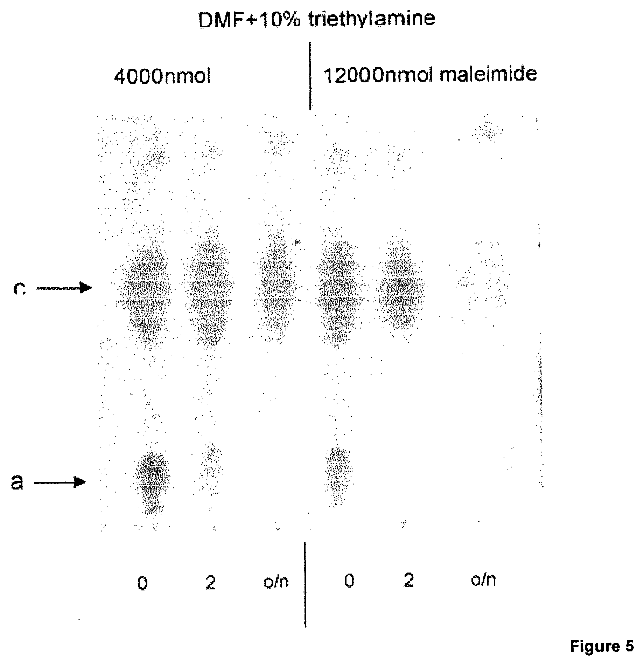

As can been seen from the TLC plate in figure 5, the amount of the c peptide mixed with a 4000 nmol equivalent of maleimide does not reduce over time, however, after an overnight reaction the amount of the c peptide does reduce when mixed with 12,000 nmol equivalent of maleimide. This is probably the result of non-specific binding of the peptide to the beads as there was three times the amount as the other reaction. Also, nothing was done to reduce non-specific binding or to wash the non-specific bound peptide from the beads when the aliquots were taken. If the whole reaction liquid was removed and the beads washed with an appropriate solvent this loss may be recovered. However, it does not stop the PST process from working it just reduced the signal strength.

Most importantly is the fact that after 2 hours the amount of 'a' peptide is reduced when reacted with 4,000 nmol of maleimide on beads and almost fully removed with 12,000 nmol. With both reactions the 'a' peptide appears to be fully removed after an overnight incubation.

The 'a' peptide appears to react with and is removed by the beads and the amount of the 'c' peptide appears to be largely unaffected by the reaction. Therefore, these observations suggested that the use of maleimide bound to polystyrene beads under non-aqueous conditions is a viable approach to isolate C-terminal peptides.

It is anticipated that a solid support derivitised with a hindered alkenyl sulphone reagent will be stable in aqueous conditions and it will be possible to isolate C-terminal peptides from an aqueous solution. Similarly a biotin reagent with a reactive functionality that comprises a hindered alkenyl sulphone should also be compatible with aqueous conditions.

### Experiments to determine how much water the maleimide beads can tolerate before hydrolysis prevents reaction with peptides

As it will probably be more practical if a certain amount of water (to aid peptide solubility etc) could be tolerated by the maleimide beads the following reactions were performed:
400 nmol of each peptide (a and b) were mixed together with bead equivalent of 12,000 nmol maleimide all dissolved in DMF with 10 % triethylamine with either 0, 10, 30 or 48% water.

The reactions were shaken at RT and samples removed as 0 and 2 hours.

Following this the peptides were separated by TLC (methanol : acetic acid : ethyl acetate 1:1:2) and ninhydrin stained to assess the amount of peptide present.

### Results

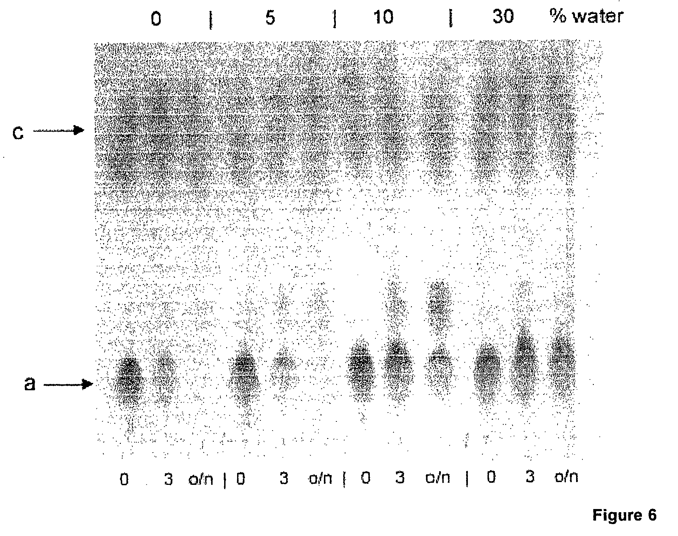

As can be seen in the TLC plate shown in figure 6, the amount of the 'a' peptide is reduced after 2 hours with 0% and 10% water but is little changed with 30% or 48%. The amount of the 'c' peptide does not appear to change apart from at 2 hours with 10% water. This can probably be explained by the fact that this particular sample did not freeze dry very well prior to TLC and was therefore applied at a greater volume causing the 'c' peptide to diffuse more.

The above results suggest that a small amount of water can be tolerated in this reaction under the conditions used. Further investigation is require to evaluate whether a greater amount of water can be tolerated under more optimised conditions

### Example 2 - Synthesis of pyridyl propenyl sulphone biotin

### Synthesis of pyridyl-1-propenylsulphone

Preparation of Pyridine-3-sulphonylchloride: 3.18g (0.02 mol) of pyridine-3-sulphonic acid (C₅H₅NSO₃) was mixed with 8.34g (0.04 mol) of PCl₅ in a dry flask. The flask was protected from moisture and heated at 130-140°C under reflux with stirring for 2 hours. The reaction mixture was then cooled. The cold solidified reaction mixture was then triturated with CHCl₃ to remove PCl₅ and POCl₃. The supernatant liquid was discarded. The triturating process was repeated using fresh CHCl₃ and the product was finally triturated with CHCl₃ saturated with hydrogen chloride. The hydrogen chloride was prepared by the slow addition of concentrated sulphuric acid (H₂SO₄) from a dropping funnel to sodium chloride in a round bottom flask. The round bottom flask was connected to the trituration reaction vessel by rubber tubing. A white powder formed, which was filtered, washed with CHCl₃ and finally dried in a vacuum. This process gave 3-pyridinesulphonylchloride-HCl (yield 3.05g, 85 %) C₅H₄NSO₂Cl, (Melting point: 141-143°C). This procedure is described by Reinhart F. E., J. Franklin. Ind. 236, 316-320 (1943).

### Preparation of Pyridine-3-(2-hydroxypropyl)sulphone

Into a boiling solution of 3.52g (0.028 mol) Na₂S0₃ and 4.36g (0.052 mol) NaHC0₃ in 50 ml water, the 3-pyridinesulphonyl chloride hydrochloride 2.828 g (0.014 mol) was added portion wise. After completion of addition, it was heated for a further 5 minutes, filtered and the filtrate evaporated to dryness. The fully pulverised residue was suspended in 100 ml of absolute dimethylformamide and heated with 1g (3mmol) of tetrabutylammonium bromide (serves as a transfer catalyst) and 2.22g (0.028 mol) of 1-chloro-2-propanol, prepared as described above. The reaction mixture was refluxed for 24 hours. After filtration of the solid, the filtrate was evaporated to dryness, and the residue oil was eluted from a silica gel column with ethyl acetate and methanol (80/20 v/v).

### Mesylation of pyridine-3-(2-hydroxypropyl)sulphone and elimination of mesylated hydroxyl to give pyridine-1-propenylsulphone

A mixture of 2.0 g (0.00995 mol) of pyridine-3-(2-hydroxypropyl) sulphone in 25 ml tetrahydrofuran (THF) and triethylamine 2.0 g (0.0199 mol) was cooled to 0°C. To this was added 2.23 g (0.0149 mol) of methane sulphonyl chloride. The reaction mixture was stirred for 6 hours at 0°C followed by stirring for 6hours at room temperature. The precipitate of triethylammonium chloride was filtered off and the solvent was evaporated. The residual oil was then treated with 1.5 g (0.0149 mol) of triethylamine and left stirring for 48 hours at room temperature. 25 ml of THF was then added, and the precipitate was filtered off. After evaporation of the solvent, the residue was eluted from a silica gel column with a solvent comprising 75 % ethyl acetate and 25 % n-hexane to afford a colourless oil, which solidified on cooling to give 1.5g of pyridine-1-propenylsulphone (83 % yield).

### Procedure for the synthesis of N-(+)-Biotin-6-amidohexyl-1-iodide

The synthesis was of N-(+)-Biotin-6-amidohexyl-1-iodide was carried out in two steps as shown in the first two steps of figure 1. In the first step D-(+)-Biotin was coupled with 6-Amino-1-hexanol to form of N-(+)-Biotin-6-amido-1-hexanol. In the second step the hydroxy group of N-(+)-Biotin-6-amido-1-hexanol was displaced by iodide.

### 1) Synthesis of N-(+)-Biotin-6-amido-1-hexanol:

Biotin was coupled to 6-amino-1-hexanol using diphenylphosphinic chloride ("Synthesis of Cyclosporin analogues". I.J. Galpin, A.Karim, A. Mohammed and A. Patel, Tetrahedron Letters vol. 28, No. 51, p. 6517-6520, 1987; "Synthetic studies of Cyclosporin Analogues". I.J. Galpin, A.Karim.A. Mohammed and A. Patel. Tetrahedron vol. 44, No. 6, p. 1783-1794, 1988) to activate the free carboxyl group of the biotin to form a mixed anhydride. 0.976g of D-(+)-Biotin (4 mmol) and 0.606g of triethylamine (6 mmol) in 20ml of distilled, dried dimethylformamide was cooled in an ice and salt bath to -5 °C. To this was added 1.416g of diphenylphosphinic chloride (6 mmol). The reaction mixture was stirred at -5 °C for 20 minutes followed by the addition of 0.702g of 6-amino-1-hexanol (6 mmol). The reaction mixture was stirred for 1 hour at 0 °C and for a further 24 hours at room temperature. The precipitated triethylammonium hydrochloride was filtered off and the solvent was removed under high vacuum. The residue obtained after evaporation of the solvent was partially purified on an ion exchange column pre-packed with a strongly basic resin (Dowex 550A OH anion exchange resin). The resin was first washed with methanol (2-bed volumes) followed by washing with 4 molar sodium hydroxide (1-bed volume) and finally by washing with aqueous methanol (20% methanol) to give pH 8-9. The crude solid residue of N-(+)-Biotin-6-amido-1-hexanol was dissolved in 5ml of methanol. The methanol solution was introduced into the column. The product eluted continuously with methanol until completion (monitored by TLC). The solvent was removed by rotary evaporation. The solid residue obtained was further purified on a silica gel column eluted with a solvent mixture of 75% ethyl acetate and 25% methanol. After evaporation of the solvents, the solid residue was recrystallised from methanol/ether to give fine needle-crystals (1.16g, 86% yield) of N- (+) Biotin-6-amido-1-hexanol (melting point: 170-172 °C). The identity of the product was confirmed by ¹H NMR, Chemical Ionisation Mass Spectrometry and microanalysis (C, H and N).

### 2) Synthesis of N-(+)-Biotin-6-amidohexyl-1-iodide:

Displacement of the hydroxy group of N- (+) Biotin-6-amido-1-hexanol by iodide with the formation of N-(+)-Biotin-6-amidohexyl-1-iodide was carried using the method of Olah et al. (J.Org.Chem **44**(8):1217, 1979) with some modifications as follows: 1.029g of N-(+)-Biotin-6-amido-1-hexanol (3 mmol) was dissolved in 15ml of acetonitrile (pre-distilled HPLC grade). The solution was then protected from moisture and purged with a continuous stream of nitrogen for 10 minutes. To this was then added 0.9g of sodium iodide (2x3 mmol) in 5ml of acetonitrile. This was followed by slow addition of chlorotrimethylsilane 0.561g (2x3 mmol) with stirring in a continuous stream of nitrogen. The formation of the product was monitored by thin layer chromatography on a silica gel developed with a solvent mixture of (75% ethyl acetate and 25% methanol). The complete disappearance of the starting material was observed after 5 hours, but the reaction was allowed to stand with stirring for 17 hours to ensure that complete displacement of the hydroxyl group was obtained. Upon completion, the reddish precipitated was filtered off and kept aside, while the filtrate was evaporated to dryness. The reddish residue obtained was added to the precipitate from the filtration. The combined solid was then dissolved completely in methanol (20 ml), and stirred with 20 ml of 10% (w/w) sodium thiosulphate until complete loss of colour of the solution was observed. 100ml of water was then added to the emulsion, which was left to stand on ice for 1 hour. The precipitate was then filtered off, washed several times with water and dried under vacuum. Re-crystallisation of the product from methanol/ether yielded 1.082g of N-(+)-Biotin-6-amidohexyl-1-iodide as a pale yellowish solid (79% yield, melting point 146-147 °C). The identity of the product was confirmed by ¹H NMR, Chemical Ionisation Mass Spectrometry and microanalysis (C, H and N).

### Synthesis of N-(+)-Biotin-6-amidohexyl-1-pyridinium-3-prop-1-en-sulphon iodide:

453mg of N- (+)-Biotin-6-amidohexyl-1-iodide (1 mmol) and 201mg of pyridyl-1-propenylsulphone (1 mmol) in 5 ml of dimethylsulphoxide (DMSO) were heated in an oil bath at 100 °C for 24 hours. The formation of the product was monitored by Thin Layer Chromatography. The DMSO was evaporated under high vacuum and the residue was then dissolved in 25 ml of water. The aqueous solution was then washed twice with chloroform. After evaporation of the water, the residue was washed twice with diethyl ether. The residue was then dissolved in methanol and evaporated to dryness. The yellowish solid obtained (252mg, 38% yield) was N-(+)-Biotin-6-amidohexyl-1-pyridinium-3-prop-1-en-sulphon iodide, also referred to as pyridyl propenyl sulphone biotin as shown in figure 1. A more pure sample was obtained later by purifying 40mg of this compound on a Sephadex column (Sephadex G15) using water as the eluent.

## Claims

1. A method for characterising a polypeptide or a population of polypeptides, which method comprises the steps of:
(a) contacting a sample comprising one or more polypeptides with a sequence specific cleavage reagent which cleaves one or more polypeptides on the C-terminal side of a lysine residue to produce peptide fragments;
(b) optionally deactivating the cleavage reagent;
(c) contacting the sample with a lysine reactive agent to cap ε-amino groups;
(d) removing those peptide fragments having capped ε-amino groups; and
(e) recovering the C-terminal peptide fragments.

2. A method according to claim 1, wherein peptide fragments having capped ε-amino groups are removed by capturing them on a solid phase and C-terminal peptides are recovered in solution.

3. A method according to claim 2, wherein the lysine reactive agent is covalently attached to a solid phase.

4. A method according to 2, wherein the peptide fragments having capped ε-amino groups are removed by affinity capture and wherein the lysine reactive agent comprises biotin and the solid phase is an avidinated solid phase.

5. A method according to any preceding claim, wherein the lysine reactive agent comprises a hindered Michael reagent.

6. A method according to any preceding claim, wherein the hindered Michael agent comprises a compound having the following structure: 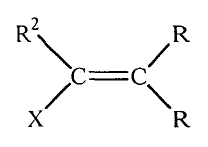 wherein X is an electron withdrawing group that is capable of stabilising a negative charge; the R groups independently comprise a hydrogen, a halogen, an alkyl, an aryl, or an aromatic group with the proviso that at least one of the R groups comprises a sterically hindering group; and the group R² comprises a hydrogen, a halogen, a hydrocarbon group, an electron withdrawing group and/or a linker capable of attachment to an affinity capture functionality or a solid phase support.

7. A method according to claim 6, wherein one R comprises a methyl or phenyl group.

8. A method according to claim 6 or claim 7 wherein at least one R comprises an electron withdrawing group.

9. A method according to any of claims 6-8, wherein at least one R comprises a cyclic or heterocylic aromatic ring or fused ring.

10. A method according to any of claims 6-9, wherein X comprises an -SO₂R¹ group, wherein R¹ comprises an alkyl group or an aryl group, including aromatic groups cyclic groups, fused cyclic groups, and heterocyclic groups.

11. A method according to claim 10, wherein R¹ comprises an electron withdrawing group.

12. A method according to claim 10 or claim 11, wherein the ring comprises a phenyl, pyridyl, naphthyl quinolyl, pyrazine, pyrimidine or triazine ring structure.

13. A method according to any of claims 6-12 wherein the X group is substituted with an electron withdrawing group.

14. A method according to claim 13, wherein the electron withdrawing group is selected from halogens, such as fluorine chlorine, bromine or iodine, and nitro and nitrile groups.

15. A method according to any of claims 6-14, wherein the X group comprises a structure capable of promoting water solubility.

16. A method according to any one of the preceding claims, wherein the cleavage agent comprises a peptidase.

17. A method according to claim 16, wherein the peptidase comprises Lys-C.

18. A method according to any one of the preceding claims, wherein the sample of step (a) comprises a sub-cellular fraction.

19. A method according to any one of the preceding claims, which further comprises preparing the sample of step (a) by liquid chromatography.

20. A method for assaying for one or more specific polypeptides in a test sample, which comprises performing a method according to any one of claims 1 to 19, wherein the sequence of the specific polypeptide is determined by assaying the resulting C-termini for a predetermined C-terminal sequence of amino acid residues.

21. A method of characterising one or more mixtures of polypeptides, which method comprises the following steps:
(a) recovering one or more C-terminal peptides from the mixtures by employing one or more of the methods as defined in any of claims 1-19;
(b) detecting the peptides by mass spectrometry.

22. A method for determining the expression profile of a sample, which method comprises characterising one or more mixtures of polypeptides according to a method as defined in claim 21.

23. A method according to claim 21 or claim 22, which method comprises determining the identity of each of the peptides detected by mass spectrometry.

24. A method according to any of claims 21-23, which method comprises identifying the quantity of each of the peptides detected by mass spectrometry.

25. A method for characterising a polypeptide or a population of polypeptides, which method comprises contacting a sample comprising one or more polypeptides with a lysine reactive agent to attach the agent to ε-amino groups, wherein the lysine reactive agent comprises a hindered Michael reagent.

26. A method according to claim 25, wherein the hindered Michael agent is a compound as defined in any of claims 6-15.

27. A compound having the following structure: 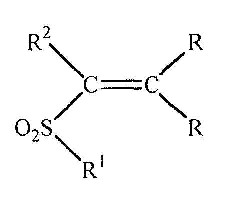 wherein R¹ comprises a pyridyl, quinolyl, pyrazine, pyrimidine or triazine ring structure and the R groups independently comprise a hydrogen, a halogen, or an alkyl or aryl group with the proviso that at least one of the R groups comprises a sterically hindering group; and the group R² comprises a hydrogen, a halogen, a hydrocarbon group, an electron withdrawing group and/or a linker capable of attachment to an affinity capture functionality or a solid phase support.

28. A compound according to claim 27, wherein at least one R group comprises a methyl or phenyl group.

29. A compound according to claim 27 or claim 28, wherein at least one R group comprises an electron-withdrawing group.

30. A compound according to claim 29 wherein at least one R group comprises a halogen atom or a halogenated alkyl group, or a phenyl ring with one or more electron withdrawing substituents.

31. A kit for characterising a polypeptide or a population of polypeptides, which kit comprises:
(a) a lysine reactive agent for capping ε-amino groups;
(b) a means for recovering or isolating C-terminal peptides;
(c) optionally an amine reactive reagent for labelling α-amino groups;
(d) optionally a sequence specific cleavage reagent for producing peptide fragments.

32. A kit according to claim 31, wherein the lysine reactive agent comprises a compound having the following structure: 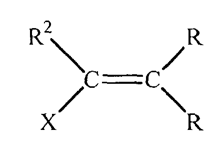 wherein X is an electron withdrawing group that is capable of stabilising a negative charge; the R groups independently comprise a hydrogen, a halogen, an alkyl, an aryl, or an aromatic group with the proviso that at least one of the R groups comprises a sterically hindering group; and the group R² comprises a hydrogen, a halogen, a hydrocarbon group, an electron withdrawing group and/or a linker capable of attachment to an affinity capture functionality or a solid phase support.

33. A kit according to claim 32, wherein the lysine reactive agent comprises a compound as defined in any of claims 27-30.

34. A kit according to any of claims 31-33, wherein the means for recovering or isolating C-terminal peptides comprises an affinity capture agent attached to the lysine reactive agent, or a solid phase covalently bound to the lysine reactive agent.

35. Use of a compound having the following structure for protecting ε-amino groups in peptides and polypeptides: 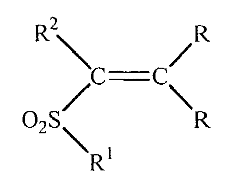 wherein R¹ comprises a pyridyl, quinolyl, pyrazine, pyrimidine or triazine ring structure and the R groups independently comprise a hydrogen, a halogen, or an alkyl or aryl group with the proviso that at least one of the R groups comprises a sterically hindering group; and the group R² comprises a hydrogen, a halogen, a hydrocarbon group, an electron withdrawing group and/or a linker capable of attachment to an affinity capture functionality or a solid phase support.

36. Use according to claim 35, wherein R¹ comprises a pyridyl, quinolyl, pyrazine, pyrimidine or triazine ring structure.

37. Use according to claim 35 or claim 36, wherein at least one R group comprises a methyl or phenyl group.

38. Use according to any of claims 35-37, wherein at least one R group comprises an electron-withdrawing group.

39. Use according to claim 38 wherein at least one R group comprises a halogen atom or a halogenated alkyl group, or a phenyl ring with one or more electron withdrawing substituents.

40. Use according to any of claims 35-39, wherein the protection is against further reaction of the ε-amino groups with Edman agents, capture agents and agents which are capable of reacting with α-amino groups.

41. Use according to claim 40, wherein the Edman agent comprises an isothiocyanate or an isocyanate, the capture agent comprises N-hydroxysuccinimidyl biotin and the agent which is capable of reacting with α-amino groups comprises acetic acid N-hydroxysuccinimide ester.
